# EUROPEAN PATENT APPLICATION

(11) **EP 4 166 672 A1**
(43) Date of publication of application: **19.04.2023**
(21) Application number: 21203021.7
(22) Date of filing: 15.10.2021
(51) Int. Cl.: C12N 15/86, C12N 15/87

(54) **A NOVEL METHOD FOR PURIFICATION OF PROTEIN- OR PEPTIDE-BASED CAPSULES**

(71) Applicant: NEUWAY Pharma GmbH, 53117 Bonn (DE)
(72) Inventor: DEMINA, Victoria, 53117 Bonn (DE); STAPF, Marcus, 53117 Bonn (DE); BONSE, Anke, 53117 Bonn (DE); KOSSMANN, Katja, 53117 Bonn (DE); DEMINA, Victoria, 53117 Bonn (DE)
(74) Representative: König Szynka Tilmann von Renesse Patentanwälte Partnerschaft mbB Düsseldorf

(57) **Abstract**

The invention relates to a method for purification of protein- or peptide-based capsules loaded with a cargo molecule from a mixture comprising said protein- or peptide-based capsules loaded with a cargo molecule and unloaded protein- or peptide-based capsules, the method comprising a step of subjecting the mixture to destabilization, preferably by adding a destabilizing agent and/or changing the physicochemical conditions, wherein the percentage of loaded protein- or peptide-based capsules is increased. The invention further relates to a composition and a Drug Delivery System obtainable by the method. The invention further relates to a Drug Delivery System obtainable by the method for use in a method of treating a neurological disorder, such as an LSD, Canavan disease or Krabbe disease.

## Description

### FIELD OF THE INVENTION

The invention relates to a method for purification of protein- or peptide-based capsules loaded with a cargo molecule from a mixture comprising said protein- or peptide-based capsules loaded with a cargo molecule and unloaded protein- or peptide-based capsules. The invention further relates to a composition and a drug delivery system obtainable by such method. The invention still further relates to said composition or drug delivery system for use in a method of treatment and/or diagnosis.

### BACKGROUND OF THE INVENTION

In general, there is a need to provide safe and effective methods to deliver a cargo molecule, for example a drug, a gene therapeutic nucleic acid or a dye, to its target site in the body for varying purposes. Particularly, if the cargo for this purpose must pass an intact physiological or biological barrier, particularly the blood brain barrier (BBB), protein- or peptide-based capsules (PPCs) are getting more acceptance as carriers for the delivery of the cargo molecule to the target site.

The use of such PPCs is particularly advantageous as the composition of the capsule's components can be chosen and respective properties, particularly regarding the tropism of the PPCs, can be achieved.

For example, such PPCs may be provided in the form of virus-like particles (VLPs). Also forms of PPCs other than VLPs are known to the person skilled in the art and can be advantageously used to deliver the cargo molecule to the target site or target system.

For the above and other reasons, PPCs may advantageously have the potential to produce safer and cheaper carriers for vaccination, gene therapy, in vivo live imaging, or many other suitable applications, particularly, if they are VLPs.

The person skilled in the art knows various ways to provide protein- or peptide-based capsules, and compositions or drug delivery systems comprising the same. Usually, the method of producing a PPC, particularly a VLP, comprises at least a step of assembly of the capsule components under formation of the capsule.

Thereby, such methods known in the art comprise further steps to load the capsules with a cargo molecule.

The WO 2020/125962, for example, discloses a method for providing a drug delivery system on the basis of VLP derived from John Cunningham virus (JCV), in particular for the delivery of a pharmaceutical product into the CNS, which has an improved efficacy.

It is known in the art that such methods of providing PPCs loaded with a cargo molecule usually result in a mixture of varying percentage of said protein- or peptide-based capsules loaded with a cargo molecule and unloaded protein- or peptide-based capsules. In other words - depending on the various parameters of process conditions, the choice of cargo molecule and composition of PPC - the loading usually is not efficient to a degree that all, or the vast majority of PPCs is successfully loaded with the cargo molecule. Hence, also unloaded PPCs are unintentionally created and comprised in the mixture.

This is particularly detrimental to a patient being treated by PPCs since the ratio of a mixture of PPCs (loaded and unloaded) can vary dramatically if loaded PPCs cannot be purified from unloaded PPCs, i.e. one therapeutic application with mixed PPCs could comprise 1% unloaded PPCs while another one could comprise 50% unloaded PPCs which deviation would not be acceptable in a clinical setting.

Furthermore, the required higher dosing of PPCs (mixture of loaded and unloaded PPCs) providing for therapeutic efficacy may induce severe side effects in patients, thus imposing a higher risk due to possible toxicity.

However, no method is known that allows increasing the percentage of loaded PPCs, i. e. a method for purification of protein- or peptide-based capsules loaded with a cargo molecule from a mixture comprising said protein- or peptide-based capsules loaded with a cargo molecule and unloaded protein- or peptide-based capsules.

### SUMMARY OF THE INVENTION

It is thus an objective of the present invention to provide a method for purification of protein- or peptide-based capsules loaded with a cargo molecule from a mixture comprising said protein- or peptide-based capsules loaded with a cargo molecule and unloaded protein- or peptide-based capsules, so that the percentage of loaded protein- or peptide-based capsules is increased. It is a further objective of the present invention to provide a composition and/or drug delivery system (DDS) obtainable by the method above. It is particularly intended to provide a DDS for use in a therapeutic application, in particular for the treatment of a lysosomal storage disease (LSD), Canavan disease and/or Krabbe disease.

It was surprisingly found by the inventors that the percentage of PPCs loaded with a cargo molecule in a mixture comprising said PPCs loaded with a cargo molecule and unloaded PPCs can be advantageously increased by a step a) of subjecting the mixture to destabilization. The inventors have surprisingly found that the mechanical and/or chemical stability of PPCs loaded with a cargo molecule is higher compared with the stability of unloaded PPCs when subjected to such destabilization.

A higher percentage of loaded PPCs in said mixture is advantageous because a higher efficacy can be achieved when a composition or drug delivery system comprising such loaded PPCs are used. In particular, a lower dose can be used. Furthermore, having unloaded PPCs in a composition or drug delivery system may interfere with the desired application, for example due to competing with the loaded PPCs.

In particular, the purification of the inventive mixture from unloaded PPCs preferably allows a therapeutic application of the loaded PPCs because only by such purification a reliable DDS can be obtained which comprises a preferably defined and equal amount of loaded PPCs in different samples.

Hence, the inventive method particularly provides for benefits in terms of safety, toxicity and therapeutically manageable dosing of PPCs being loaded with preferably therapeutically applicable cargo to be used as DDS, particularly in gene therapeutic applications.

The method according to the present invention for purification of protein- or peptide-based capsules loaded with a cargo molecule from a mixture comprising said protein- or peptide-based capsules loaded with a cargo molecule and unloaded protein- or peptide-based capsules, comprises at least a step a) of subjecting the mixture to destabilization, preferably by adding a destabilizing agent and/or changing the physicochemical conditions, wherein the percentage of loaded protein- or peptide-based capsules is increased.

The increased percentage of PPCs loaded with a cargo molecule according to the present invention may be demonstrated by various methods known in the art. The person skilled in the art will thereby acknowledge that the method of choice can be selected depending on the particular PPC and the cargo molecule.

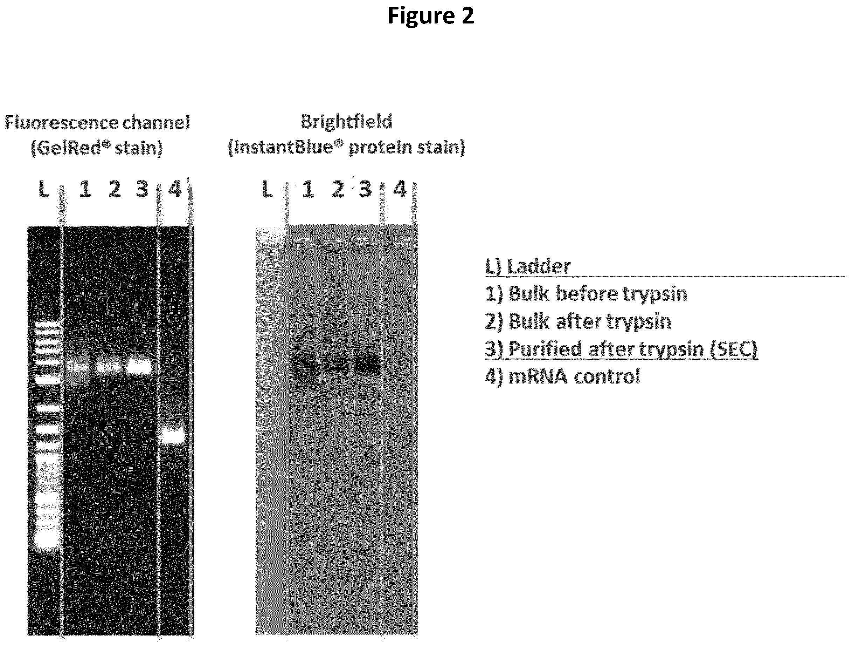

The increased percentage of PPCs loaded with an mRNA molecule as a cargo molecule obtainable by the method according to the invention is herein demonstrated by the increased and purified band on an agarose gel after agarose gel electrophoresis (AGE) (Example 2 below, Fig. 2).

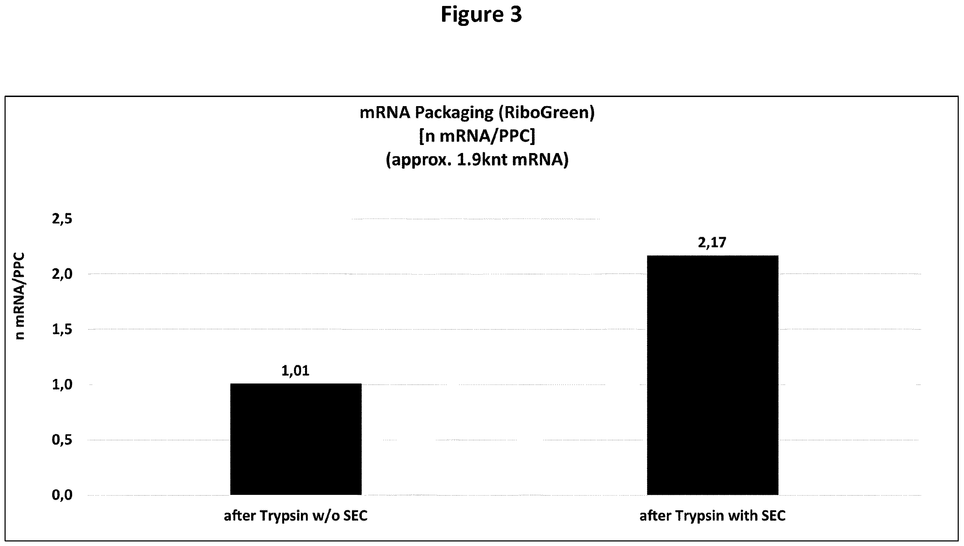

Additionally, the increased percentage of PPCs loaded with an mRNA molecule as a cargo molecule obtainable by the method according to the invention is herein demonstrated by the increased ratio of number of mRNA molecules per PPC in a RiboGreen^{™} Assay (Example 3 below, Fig. 3).

Additionally or alternatively, the increased percentage of PPCs loaded with an mRNA molecule or plasmid, respectively, as a cargo molecule obtainable by the method according to the invention can also be demonstrated by the number and purity of PPCs loaded with an mRNA molecule or plasmid, respectively, in a Transmission electron microscopy (TEM) analysis (Example 5 below, Fig. 6, and Examples 6 and 7 below, Figs. 7 and 8).

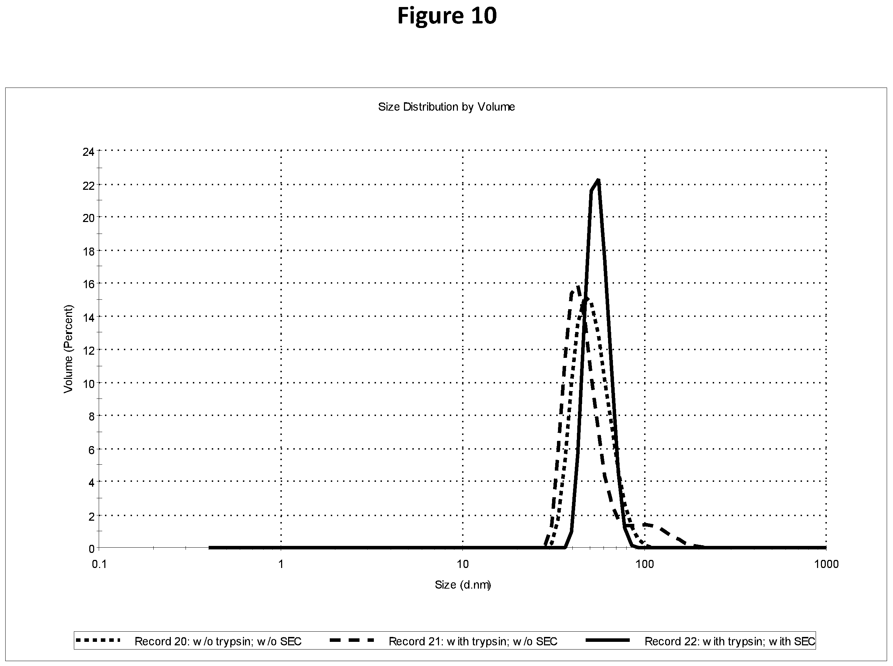

Additionally or alternatively, the improved percentage of PPCs loaded with an antibody molecule as a cargo molecule obtainable by the method according to the invention can also be demonstrated by the peak characteristics, particularly the sharpness and/or size characteristics of a peak of PPCs loaded with an antibody molecule in a dynamic light scattering (DLS) analysis (Example 9 below, Fig. 10).

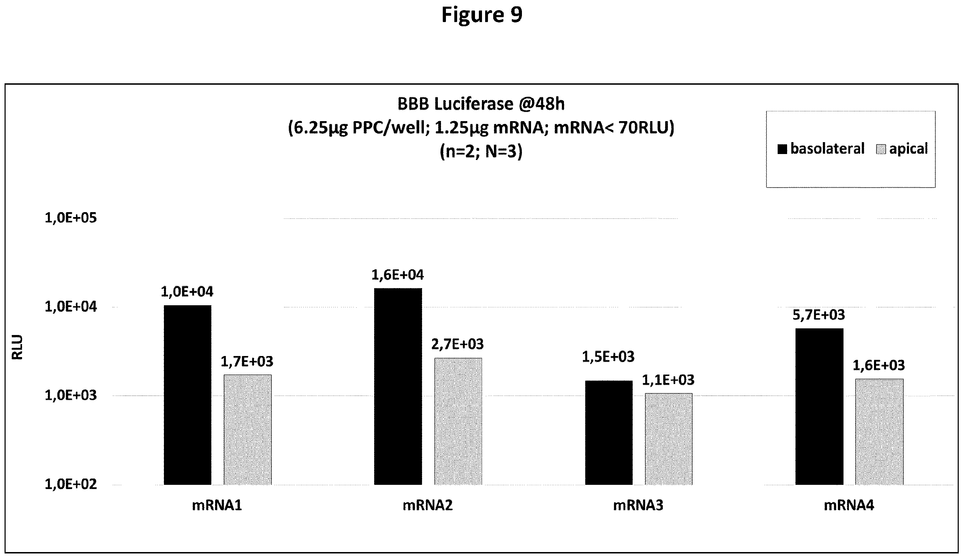

Examples 8 and 11 furthermore give evidence that the PPCs obtained by the method according to the present invention efficiently cross the BBB (Examples 8 and 11 below, Figs. 9, 12 and 13). Therewith, evidence is provided, that the PPCs can be used as a composition and/or a drug delivery system for the transport of a cargo molecule, particularly a pharmaceutically acceptable product, a dye and/or an inorganic compound into the CNS.

### BRIEF DESCRIPTION OF THE FIGURES

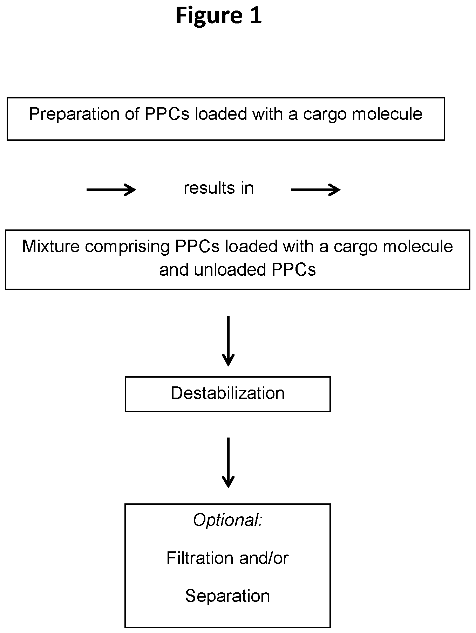

Fig. 1 shows a schematic flow chart of a method for producing a mixture comprising PPCs loaded with a cargo molecule and unloaded PPCs further comprising an embodiment of the method for purification according to the present invention;
Fig. 2 shows an Agarose Gel Electrophoresis (AGE) analysis for analysing the presence of PPCs and verifying co-localization of protein and encapsulated mRNA cargo molecules;
Fig. 3 shows the quantification of the amount of encapsulated mRNA cargo molecules with a RiboGreen^{™} Assay after trypsin digestion without and with subsequent SEC treatment;

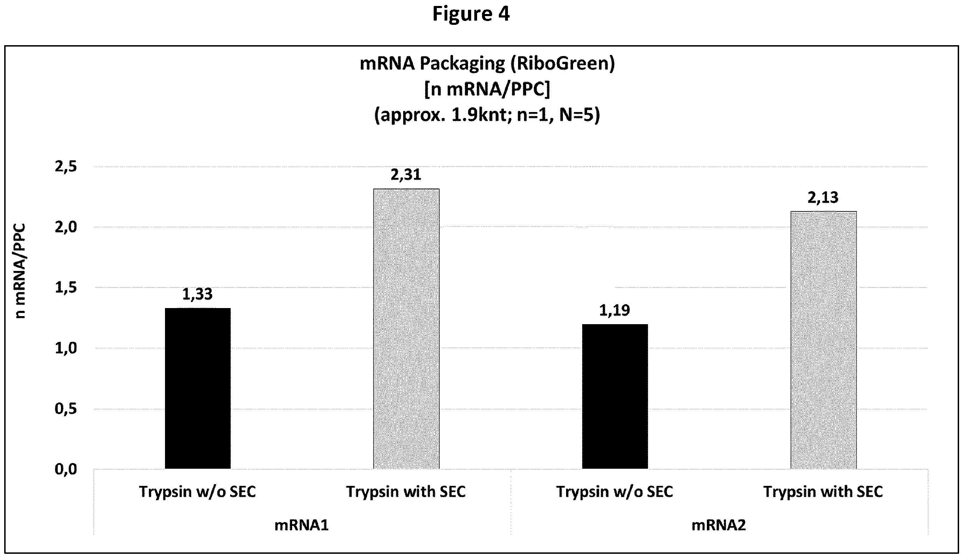

Fig. 4 shows the quantification of the amount of two further encapsulated mRNA cargo molecules with a RiboGreen^{™} Assay after trypsin digestion without and with subsequent SEC treatment;

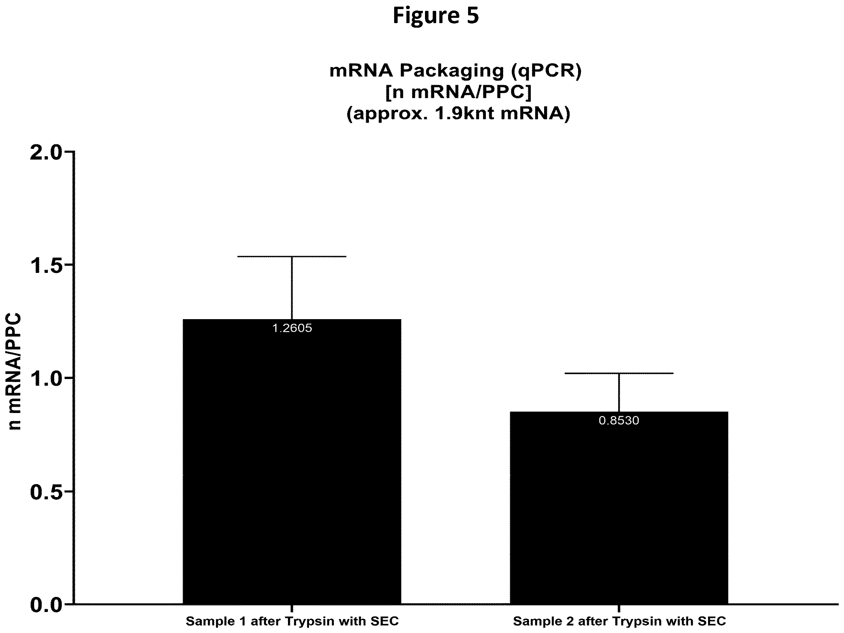

Fig. 5 shows the quantification of the amount of encapsulated mRNA cargo molecules with a qPCR after trypsin digestion with subsequent SEC treatment;

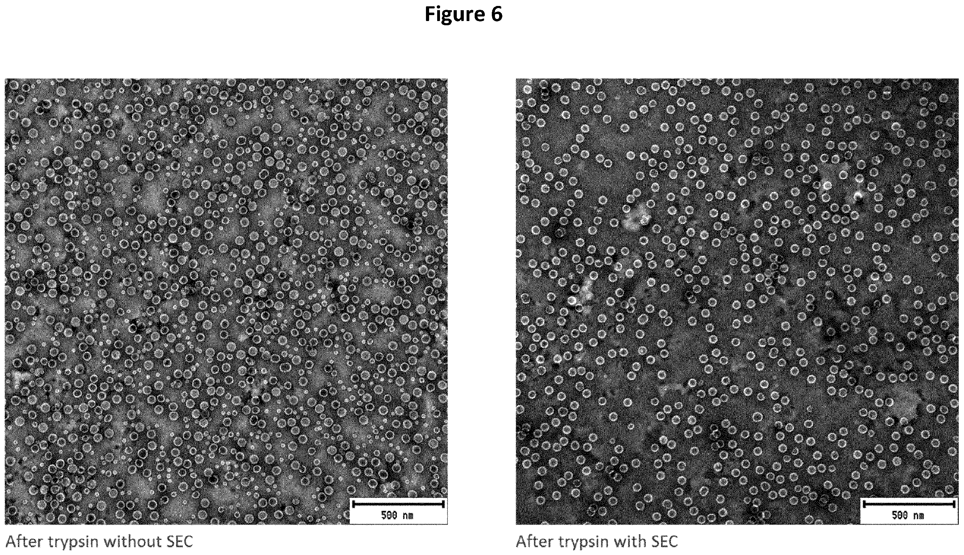

Fig. 6 shows TEM images of a mixture comprising PPCs loaded with mRNA molecule as a cargo molecule and unloaded PPCs after a trypsin destabilization step and with and without subsequent application of a step b2) of SEC separation;

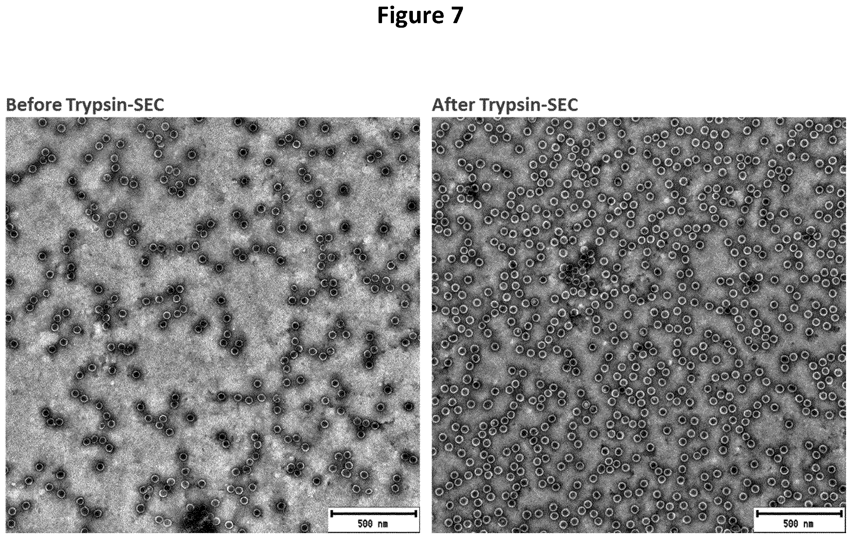

Fig. 7 shows TEM images of a mixture comprising PPCs loaded with a plasmid molecule (pNL) as a cargo molecule and unloaded PPCs before a trypsin digestion step and without SEC (left) and after a trypsin digestion step and SEC (right);

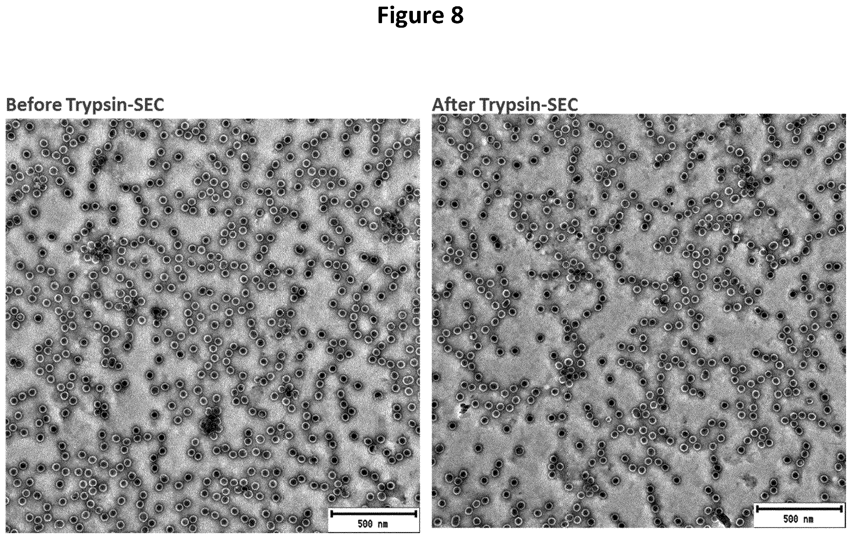

Fig. 8 shows TEM images of a mixture comprising PPCs loaded with a plasmid molecule (pGL4.51) as a cargo molecule and unloaded PPCs before a trypsin digestion step and without SEC (left) and after a trypsin digestion step and SEC (right);
Fig. 9 shows Luciferase activity measured in glioblastoma cells transfected with VLP loaded with Luciferase mRNA in apical or basolateral subcellular localization (BBB model);
Fig. 10 shows a DLS analysis showing the size and dispersity of a mixture comprising a PPC loaded with an antibody as a cargo molecule and unloaded PPCs prior and after applying the method according to the present invention;

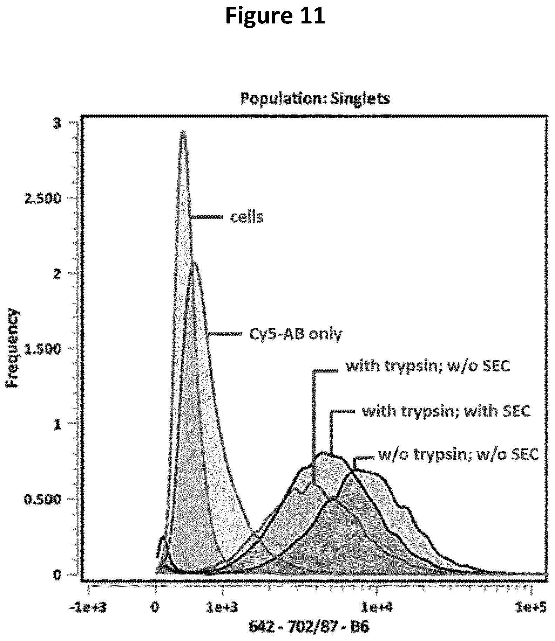

Fig. 11 shows a Flow Cytometry analysis visualizing the attachment of antibodies VLPs are loaded with to cells using a mixture comprising a PPC loaded with an antibody as a cargo molecule and unloaded PPCs prior and after applying the method according to the present invention;

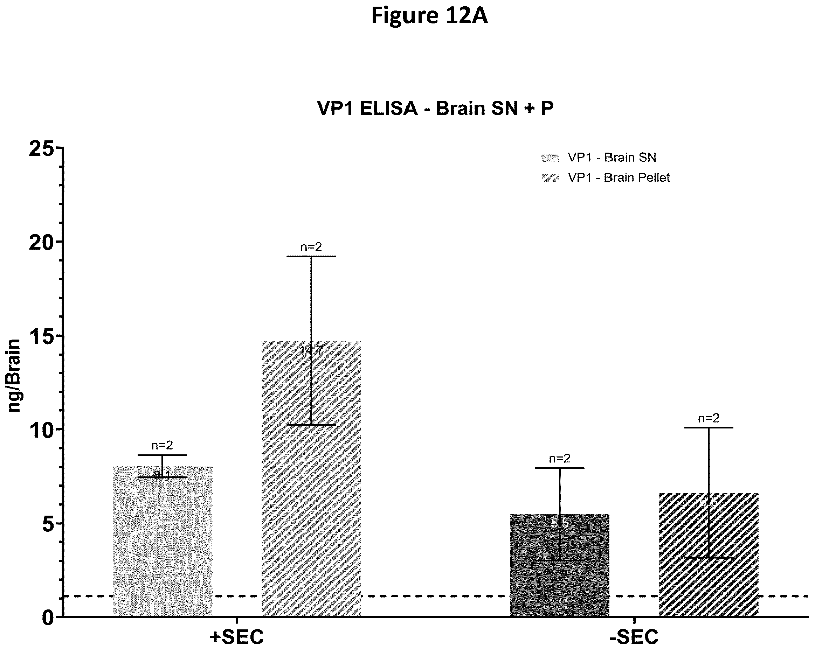

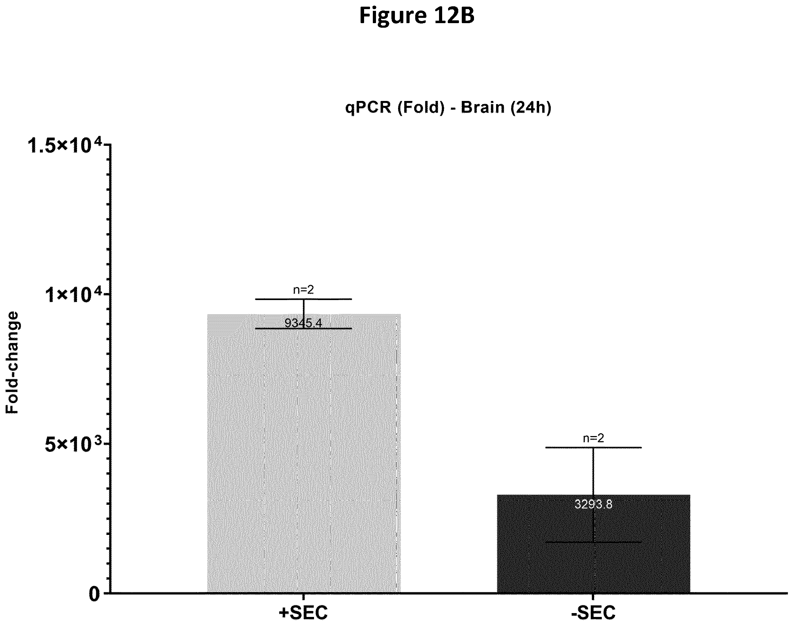

Figs. 12A and 12B show the results of an ELISA assay (Fig. 12A) and a qPCR assay (Fig. 12B), respectively, of brain samples of mice injected with a mixture comprising PPCs loaded with mRNA as a cargo molecule and unloaded PPCs before and after application of the method according to the present invention.

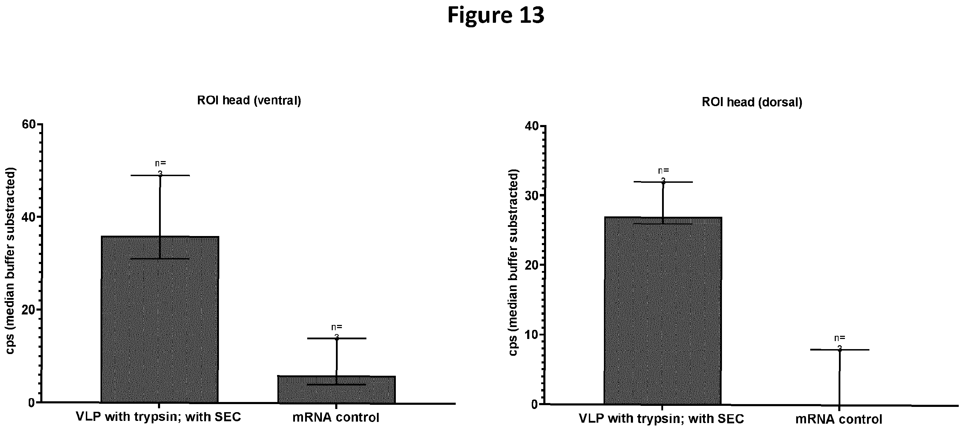

Fig. 13 shows ex vivo Luciferase activity in heads (ventral and dorsal) of mice injected with a mixture comprising PPCs loaded with mRNA as a cargo molecule and unloaded PPCs (compared with an mRNA control).

### DETAILED DESCRIPTION OF THE INVENTION

In a first aspect of the invention, the invention relates to a method for purification of protein- or peptide-based capsules loaded with a cargo molecule from a mixture comprising said protein- or peptide-based capsules loaded with a cargo molecule and unloaded protein- or peptide-based capsules.

The method according to the present invention thereby comprises at least
a step a) of subjecting the mixture to destabilization, preferably by adding a destabilizing agent and/or changing the physicochemical conditions, wherein the percentage of loaded protein- or peptide-based capsules is increased.

The use of protein- or peptide-based capsules (PPC), such as virus-like particles (VLP), is well known in the art and various methods exist to produce mixtures comprising such PPCs loaded with the cargo molecule. Thereby, the composition, and particularly the chosen peptides or proteins of the PPC, as well as the cargo molecule can be selected from a broad range of molecules to best fulfill the desired purpose.

Thereby it shall be understood that protein- or peptide-based capsules as understood herein, preferably refer to any three-dimensional structure of a protein and/or peptide layer that encapsulates an interior. The PPCs according to the present invention may thus be provided in the form of capsules. Particularly considered herein are PPCs in the form of virus-like particles (VLPs).

A "virus-like particle" as used herein refers preferably to three-dimensional capsule structures of molecules that closely resemble viruses, but are non-infectious because they contain no viral genetic material. Thereby VLPs preferably comprise at least one type of viral structural protein, such as VP1, which allows assembly, particularly self-assembly, into the virus-like capsule structure.

A "virus-like particle" (VLP) as used herein in the context of the present invention, is preferably further defined as a replication-deficient particle with a hull (also termed capsid) composed of viral structural proteins or modified viral structural proteins or proteins derived from viral structural proteins. The VLP according to the examples as disclosed below is preferably derived from JCV, i.e. its hull is composed of viral structural proteins or modified viral structural proteins or proteins derived from viral structural proteins VP1, VP2 and VP3 from JCV, in particular from VP1.

A method for providing a drug delivery system comprising a virus-like particle (VLP) derived from John Cunningham virus (JCV) is for example described in WO 2020/125962 or WO 2021/074123, which disclosures and in particular which methods are incorporated herein by reference.

Possible sequences of VP1 are also disclosed in WO 2020/125962, which are also incorporated herein by reference.

The term "VP1" according to the invention also encompasses fractions of the native VP1. Preferably, said fractions of VP1 comprise at least acids 32 to 316 of the amino acid sequence according to SEQ ID NO: 1 or a derivative thereof having an identity with the amino acid sequence from amino acid position 32 to 316 of SEQ ID NO: 1 of at least 80%, more preferably at least 85%, more preferably at least 90%, more preferably at least 95%, more preferably at least 97%, more preferably at least 98% or at least 99% over this sequence.

In a preferred embodiment of the invention the VP1 has an amino acid sequence which is at least 80 %, more preferably at least 85 %, more preferably at least 90 %, more preferably at least 95 %, more preferably at least 99 % identical to the amino acid sequence according to SEQ ID NO: 1 over its entire length. In a most preferred embodiment of the invention, the VP1 has an amino acid sequence which is identical to the amino acid sequence according to SEQ ID NO: 1.

In another embodiment of the invention the VP1 has an amino acid sequence which is at least 80 %, more preferably at least 85 %, more preferably at least 90 %, more preferably at least 95 %, more preferably at least 99 % identical to the amino acid sequence according to SEQ ID NO: 3 over its entire length. In one embodiment, the amino acid sequence of VP1 is identical to the amino acid sequence of SEQ ID NO: 3.

In one embodiment, the nucleotide sequence of the VP1 protein is at least 70 %, more preferably at least 80 %, more preferably at least 90 % identical to the nucleotide sequence of SEQ ID NO: 2 over its entire length, preferably is the nucleotide sequence of SEQ ID NO: 2.

In another embodiment of the invention the nucleotide sequence of the VP1 protein is at least 70 %, more preferably at least 80 %, more preferably at least 90 % identical to the nucleotide sequence of SEQ ID NO: 4 over its entire length. In one embodiment, the nucleotide sequence of the VP1 protein is identical to the nucleotide sequence of SEQ ID NO: 4.

The sequences are depicted below.

Amino acid and nucleotide sequences of the VP1 protein.

| **Protein** / **DNA** | **Sequence** | **Source** | **SEQ ID NO:** |
|---|---|---|---|
| **Protein** | MAPTKRKGEPKDPVQVPKLLIRGGVEVL EVKTGVDSITEVECFLTPEMGDPDEHLR GFSKSISISDTFESDSPNRDMLPCYSVAR IPLPNLNEDLTCGNILMWEAVTLKTEVIG VTSLMNVHSNGQATHDNGAGKPVQGTS FHFFSVGGEALELQGVVFNYRTKYPDGT IFPKNATVQSQVMNTEHKAYLDKNKAYP VECWVPDPTRNENTRYFGTLTGGENVP PVLHITNTATTVLLDEFGVGPLCKGDNLY LSAVDVCGMFTNRSGSQQWRGLSRYFK VQLRKRRVKNPYPISFLLTDLINRRTPRV DGQPMYGMDAQVEEVRVFEGTEELPG DPDMMRYVDKYGQLQTKML | derived from JCV | 1 |
| **DNA** | ATGGCTCCCACCAAGCGCAAGGGCGA GCCCAAGGACCCCGTGCAAGTGCCCA AGCTGCTGATCCGTGGTGGTGTCGAG GTGCTGGAAGTCAAGACCGGCGTGGA CTCCATTACCGAGGTGGAGTGCTTCCT CACCCCCGAGATGGGTGACCCTGACG AGCACCTGAGGGGCTTCTCCAAGTCC ATCTCCATCTCCGACACCTTCGAGTCC GACTCCCCCAACCGTGACATGCTGCC CTGCTACTCCGTGGCTCGTATCCCCCT GCCCAACCTGAACGAGGACCTGACTT GCGGCAACATCCTGATGTGGGAGGCT GTGACCCTCAAGACCGAGGTCATCGG CGTGACTTCCCTGATGAACGTGCACTC CAACGGCCAGGCTACCCACGACAACG GTGCTGGCAAGCCCGTGCAGGGAACC TCCTTCCACTTCTTCTCCGTGGGTGGC GAGGCTCTGGAACTCCAGGGCGTGGT GTTCAACTACCGTACCAAGTACCCCGA CGGCACCATCTTCCCCAAGAACGCTAC TGTGCAGTCCCAAGTGATGAACACCGA GCACAAGGCTTACCTGGACAAGAACAA GGCCTACCCCGTGGAGTGCTGGGTGC CCGACCCCACCCGTAACGAGAACACC CGTTACTTCGGCACCCTGACCGGTGG AGAGAACGTGCCCCCCGTGCTGCACA | derived from JCV | 2 |
| | TCACCAACACCGCTACCACCGTGCTGC TGGACGAGTTCGGTGTCGGTCCCCTG TGCAAGGGCGACAACCTGTACCTGTC CGCTGTGGACGTGTGCGGCATGTTCA CCAACCGTTCCGGTTCCCAGCAGTGG CGTGGCCTGTCCCGCTACTTCAAGGT GCAGCTGCGCAAGCGTCGTGTGAAGA ACCCCTACCCTATCTCCTTCCTGCTGA CCGACCTGATCAACCGTCGTACCCCTC GTGTGGACGGCCAGCCCATGTACGGC ATGGACGCTCAGGTGGAAGAGGTCCG CGTGTTCGAGGGCACCGAGGAATTGC CCGGCGACCCCGACATGATGCGTTAC GTGGACAAGTACGGCCAGCTCCAGAC CAAGATGCTGTAA | | |
| **Protein** | MAPTKRKGERKDPVQVPKLLIRGGVEVL EVKTGVDSITEVECFLTPEMGDPDEHLR GFSKSISISDTFESDSPSKDMLPCYSVAR IPLPNLNEDLTCGNILMWEAVTLKTEVIG VTSLMNVHSNGQAAHDNGAGKPVQGTS FHFFSVGGEALELQGVVFNYRTKYPDGT IFPKNATVQSQVMNTEHKAYLDKNKAYP VECWVPDPTRNENTRYFGTLTGGENVP PVLHITNTATTVLLDEFGVGPLCKGDNLY LSAVDVCGMFTNRSGSQQWRGLSRYFK VQLRKRRVKNPYPISFLLTDLINRRTPRV DGQPMYGMDAQVEEVRVFEGTEELPG DPDMMRYVDRYGQLQTKML | wildtype JCV (AFH57194.1) | 3 |
| **DNA** | ATGGCCCCAACAAAAAGAAAAGGAGAA AGGAAGGACCCCGTGCAAGTTCCAAA ACTTCTTATAAGAGGAGGAGTAGAAGT TCTAGAAGTTAAAACTGGGGTTGACTC AATTACAGAGGTAGAATGCTTTTTAACT CCAGAAATGGGTGACCCAGATGAGCA TCTTAGGGGTTTTAGTAAGTCAATATCT ATATCAGATACATTTGAAAGTGACTCC CCAAATAGGGACATGCTTCCTTGTTAC AGTGTGGCCAGAATTCCACTACCCAAT CTAAATGAGGATCTAACCTGTGGAAAT ATACTCATGTGGGAGGCTGTGACCTTA AAAACTGAGGTTATAGGGGTGACAAGT TTGATGAATGTGCACTCTAATGGGCAA GCAACTCATGACAATGGTGCAGGGAA | wildtype JCV (J02226) | 4 |
| | GCCAGTGCAGGGCACCAGCTTTCATTT TTTTTCTGTTGGGGGGGAGGCTTTAGA ATTACAGGGGGTGCTTTTTAATTACAG AACAAAGTACCCAGATGGAACAATTTT TCCAAAGAATGCCACAGTGCAATCTCA AGTCATGAACACAGAGCACAAGGCGTA CCTAGATAAGAACAAAGCATATCCTGT TGAATGTTGGGTTCCTGATCCCACCAG AAATGAAAACACAAGATATTTTGGGAC ACTAACAGGAGGAGAAAATGTTCCTCC AGTTCTTCATATAACAAACACTGCCACA ACAGTGTTGCTTGATGAATTTGGTGTT GGGCCACTTTGCAAAGGTGACAACTTA TACTTGTCAGCTGTTGATGTCTGTGGC ATGTTTACAAACAGGTCTGGTTCCCAG CAGTGGAGAGGACTCTCCAGATATTTT AAGGTGCAGCTAAGGAAAAGGAGGGT TAAAAACCCCTACCCAATTTCTTTCCTT CTTACTGATTTAATTAACAGAAGGACTC CTAGAGTTGATGGGCAGCCTATGTATG GCATGGATGCTCAAGTAGAGGAGGTTA GAGTTTTTGAGGGAACAGAGGAGCTTC CAGGGGACCCAGACATGATGAGATAC GTTGACAAATATGGACAGTTGCAGACA AAAATGCTGTAA | | |

In a preferred embodiment of the invention, the VP1 has an amino acid sequence which is at least 90 % identical to the amino acid sequence according to SEQ ID NO: 1 over its entire length.
In a preferred embodiment of the invention, the nucleotide sequence of the VP1 protein is at least 80 % identical to the nucleotide sequence of SEQ ID NO: 2 over its entire length.

The PPC according to the invention can furthermore comprise in the capsule one or several additional heterologous proteins, i.e. proteins which are or are not identical. Particularly, in embodiments referring to VLPs as PPCs the proteins may be identical to or derived from a JCV protein. For example, a heterologous protein can be anchored in the capsule or capsid, i.e. at least part of this protein being preferably accessible from the outside. In principle any protein is suitable as such a heterologous protein as long as the heterologous protein can be incorporated into the capsid and does not interfere substantially with the assembly of the PPC or VLP, respectively.

A "peptide" according to the present invention may preferably be composed of any number of amino acids of any type, e.g. naturally occurring or modified amino acids, which preferably are linked by peptide bonds. In particular, a peptide comprises preferably at least 3 amino acids, more preferably at least 5, at least 7, at least 9, at least 12 or at least 15 amino acids. There is no upper limit for the length of a peptide. However, preferably a peptide according to the invention does not exceed a length of 500 amino acids, preferably 400, 300, 250, 200, 150 or 120 amino acids. A peptide exceeding about 10 amino acids may also be termed a "polypeptide". A "protein" according to the present invention may for example be composed of at least 100 amino acids but also shorter proteins are known. Hence, the lengths of a "peptide" and a "protein" may overlap.

The term "cargo" as used herein, preferably refers to any molecule which the PPC is loaded with. Thereby, the term "cargo" preferably refers to the molecule that exploits the intended effect of the application of the PPC at the target site.

The PPCs are for example loaded with the cargo molecule in subsequent assembly, disassembly and re-assembly steps. Thereby, eventually also unloaded PPCs are created, where the PPC is not successfully loaded with the cargo molecule. A high percentage of unloaded PPCs in the mixture is thereby disadvantageous as the percentage of loaded PPCs vice versa is low and thus the efficacy of the cargo molecule might be disadvantageously reduced.

The term "loaded PPCs" as referred to herein, preferably refers to PPCs loaded with a cargo molecule. In contrast thereto, the term "unloaded PPCs" as referred to herein, preferably refers to PPCs not loaded with a cargo molecule.

"Loading" thereby, preferably means any process which leads to the association of PPC and cargo, e.g. by osmotic shock or by assembly of capsule proteins or peptides, such as VP1 or VP1 pentamers into VLP, together with the cargo. "Loaded PPCs" are the PPCs resulting from such process.

Additionally, or alternatively, the term "loaded PPCs" as used herein, preferably refers to a status of a PPC, wherein the cargo molecule is associated with the PPC. Thereby, such association of the cargo molecule and the PPC may preferably comprise any form of association suitable to deliver the cargo molecule to the intended target site of the PPC application, allowing to exploit the desired effect of the cargo molecule. More preferably, the term "loaded" as used herein refers to a cargo molecule being attached to, associated with or fully incorporated into the PPC. Additionally, in the context of the invention, the expression that the "PPC comprises the cargo" is preferably used synonymously with the expression that the PPC is "loaded with" or "associated with" the cargo. The loading and association of the PPC and the cargo can be the result of "loading" or "packing" the PPC with the cargo, particularly as described in WO 2020/125962 and WO 2021/074123.

Accordingly, a mixture comprising said protein- or peptide-based capsules loaded with a cargo molecule and unloaded protein- or peptide-based capsules results from said manufacturing processes, and also results in the desire to increase, and thereby improve, the percentage of loaded versus unloaded PPCs. In other words, the percentage of loaded PPCs in the mixture is increased.

Thereby, the term "percentage of loaded PPCs" as used herein, preferably refers to the percentage of loaded PPCs of all present PPCs in the mixture, and preferably an increase refers to a comparison of said percentage to the percentage of PPCs loaded with a cargo molecule of all present PPCs (loaded and unloaded) in a mixture not subjected to the method according to the present invention or before the method according to the present invention.

The inventors have surprisingly found that the percentage of loaded PPCs in a mixture can be increased, if said mixture is in said step a) subjected to destabilization.

A common method to determine mRNA or DNA amount per PPC would be RiboGreen^{™} assay or qPCR as exemplified below. The percentage of loaded PPCs of all present PPCs in the mixture can be determined by any method known by the person skilled in the art, for example by analytical ultracentrifugation which allows separating the fractions of unloaded PPC and loaded PPC. This method for example also allows separating fractions with different amounts of mRNA. Another method would be a microscopic method which visualizes the cargo, for example the RNA or DNA, for example electron microscopy. Thereby, it can be directly visualized which PPCs comprise cargo and which PPCs do not comprise cargo so that a percentage according to the present invention can be calculated.

The term "destabilization" as used herein, preferably refers to a loss of stability of a PPC, and more particularly to a reduced stability and/or impairment of the 3D structure of a PPC. Additionally or alternatively, the term "destabilization" refers to a treatment step applied according to the method of the present invention to a mixture comprising loaded and unloaded PPCs, resulting in a loss of stability of the unloaded PPCs with preference. Thereby, the 3D structure and/or the association or binding of said PPCs, and particularly the capsule components thereof maybe be affected such that the capsules may most preferably disassemble. For example, an unloaded PPC may be destabilized when subjected to a protease digestion, for example by addition of trypsin.

It is to be understood that thereby basically any means suitable to selectively destabilize unloaded PPCs but not loaded PPCs, is applicable.

The method according to the present invention preferably does not negatively impact the purpose of the PPCs, for example their desired tropism, efficacy to cross physiological and biological barriers, as well as the efficacy and integrity of the cargo molecule itself.

In one embodiment the step a) of subjecting the mixture to destabilization is carried out by the addition of a destabilization agent to the mixture and preferably a subsequent incubation with said destabilization agent. Additionally, or alternatively, the mixture can be subjected to a destabilization step a) by changing the physicochemical conditions of the mixture.

As used herein, the terms "add" or "addition" preferably refer to the addition of a respective component, more preferably comprise a subsequent incubation step, i.e. time and temperature condition, preferably to carry out the respective intended chemical or biochemical reaction. Said incubation step can be preferably stopped by methods and means known in the art, after an intended time span and/or efficacy of the incubation reaction.

In both ways, if adding a destabilization agent and/or changing the physicochemical conditions, the step a) of destabilization the result is that the percentage of loaded PPCs of all present PPCs is increased by the method according to the present invention compared to the percentage of PPCs loaded with a cargo molecule of all present PPCs (loaded and unloaded) in a mixture not subjected to the method according to the present invention.

As a result of applying the method according to the present invention to a mixture comprising said PPCs loaded with a cargo molecule and unloaded PPCs, the percentage of loaded PPCs may advantageously be increased to about 50%, preferably 60 %, preferably 70 %, preferably 80 %, preferably 90 %, preferably to about 100% of loaded PPCs of all PPCs in the mixture. Preferably, the advantageous method achieves an increased percentage of loaded PPCs of all PPCs in the mixture of at least 80%, preferably at least 85%, more preferably at least 90%, still more preferably of at least 95%, and most preferably of at least 99%.

It is important to note that in subsequent steps of the method further improvements to the purity of loaded PPCs may be carried out. Thereby, the steps are preferably but not necessarily carried out in the given order.

In a preferred embodiment of the method according to the present invention the method further comprises a step b1) of filtrating.

This step may be particularly useful to remove material from the mixture, which is no PPCs, for example non-capsule material, such as aggregates resulting from previous steps, like nuclease, e. g. Benzonase^{®} treatment, or the like. Thereby, such step of filtrating can comprise sterile filtration, dialysis, centrifugation, spin columns, in particular with immobilized nucleases and/or proteases, beads, in particular with subsequent pull-down assay, and/or cross-flow filtration or the like. Sterile filtration is preferred.

The person skilled in the art knows that a pore size for a filtrating method depends on the particular PPC and that a specific pore size may be chosen accordingly.

In a preferred embodiment of the method according to the present invention additionally or alternatively to the step b1) of filtrating, the method further comprises a step b2) of separating.

Preferably, step b2) of separating is selected from the group comprising filtration, ultrafiltration, e.g. dead-end filtration with MWCO, and/or cross flow filtration, chromatography, e.g. preparative chromatography, size-exclusion-chromatography (SEC), and/or affinity chromatography, and dynamic light scattering (DLS), preferably selected from the group comprising cross flow filtration and size-exclusion-chromatography (SEC). SEC is most preferred.

Such step b2) of separating, may be particularly useful to separate loaded PPCs from destabilized unloaded PPCs and/or any debris resulting from the destabilization step a) according to the inventive method.

In a particularly preferred embodiment, the method according to the present invention comprises a step b1) of filtrating and a step b2) of separating after a step a) of destabilization. Preferably, the step b1) of filtrating is performed by sterile filtration and the step b2) of separating is performed by SEC. Hence, in a preferred embodiment, step a) is performed by addition of trypsin, subsequently step b1) is performed by sterile filtration and subsequently step b2) is performed by SEC.

The step of applying SEC may thereby be of further advantage as it may remove protease, such as trypsin, residues from the sample. Subsequently, loaded PPCs may further be concentrated via a VivaSpin^{®} (MWCO50kDa) centrifugation step and if desired stored at for example -80°C until further use. The VivaSpin^{®} centrifugation step thereby advantageously removes small fragments, which may result after SEC or sample concentration.

The term "purification" in the context of the present invention thereby preferably refers to the selective enrichment or increase of percentage of loaded PPCs. In one embodiment, the term "purification" refers to the selective enrichment or increase of percentage of loaded PPCs and subsequent isolating or separating of the loaded PPCs from unloaded PPCs and any debris resulting from destabilization of unloaded PPCs.

The term "chromatography" as used herein, preferably refers to a method which permits the separation of a mixture of substances by distributing the individual components thereof between a stationary phase and a mobile phase. In particular, chromatography refers to a method of purifying a substance by first binding and enriching the substance of interest to the stationary phase before eluting it in a second step (bind-and-elute mode of chromatography) or by binding impurities to the stationary phase and increasing the purity of the molecule of interest in the flow-through (flow-through mode).

Chromatography can be grouped according to the basis of the interaction of the analyte comprised in the mobile phase with the stationary phase. Preferred types of chromatography according to the invention encompass "reversed phase" chromatography, "ion-exchange" chromatography, "affinity" chromatography, or size exclusion chromatography (SEC). Among ion-exchange chromatography, the purification method can be further separated based on the charge present in the stationary phase into cation-exchange chromatography (CEX), in which the stationary phase has a negative charge, thus retaining positively charged molecules, and anion exchange chromatography (AEX), in which the stationary phase has a positive charge, thus retaining negatively charged molecules.

In particular, chromatography may be used to separate loaded PPCs from destabilized unloaded PPCs and/or any debris resulting from the destabilization step a) according to the inventive method.

In a still further preferred embodiment of the method according to the present invention the destabilizing agent is a protease and/or a destabilization agent inducing a change of pH of the mixture.

Such treatment with a destabilization agent, particularly a protease, preferably trypsin, may advantageously enhance the specific degradation of unloaded protein- or peptide-based capsules in the mixture.

Particularly, a treatment with a protease as a destabilization agent may advantageously result in a specific destabilization of unloaded capsules, if the loaded capsules are more stable to such protease treatment.

In such step a) according to the present invention, of subjecting the mixture to destabilization, preferably by adding a destabilizing agent, particularly at least 10%, preferably at least 20%, more preferably at least 30%, most preferably at least 50%of the total amount of unloaded PPCs are destabilized, i. e. the 3D structure of unloaded PPCs is impaired

The destabilization of unloaded PPCs thereby may also result from the change of pH in the mixture, particularly by increasing the pH or reducing the pH. The pH depends on the PPC and its optimum pH when loaded. The pH for destabilization preferably differs from the optimum pH of the loaded PPC.

A change of pH thereby may result in a specific destabilization of unloaded capsules, if the loaded capsules are more stable to such pH change.

In such step a) according to the present invention, of subjecting the mixture to destabilization, preferably by adding a destabilization agent inducing a change of pH of the mixture particularly at least 10%, preferably at least 20%, more preferably at least 30%, most preferably at least 50% of the total amount of unloaded PPCs are destabilized, i. e. the 3D structure of unloaded PPCs is impaired.

Notably, the inventors have surprisingly found that basically any adding of a destabilization agent may result in the destabilization of unloaded PPCs, while loaded PPCs prove to be more stable also to the impact of the destabilization agent, particularly protease or the change of pH in the mixture. Thereby, the increased susceptibility of unloaded PPCs versus loaded PPCs to the respective destabilization agent can be determined with standard measures known by the person skilled in the art.

In a still further preferred embodiment of the method according to the present invention changing the physicochemical conditions is selected from the group comprising changing the physicochemical condition by mechanical treatment, particularly centrifugation or vortex, filtration with pressure, chromatography, particularly SEC with pressure, by change of temperature, particularly by heat denaturation, heat-shock treatment, preferably at temperatures of at maximum 96°C, or freeze-thaw treatment, by acoustic treatment, particularly with ultrasound, by osmotic treatment, particularly osmotic shock and pressure, by change of pH, particularly by adding acids or bases, and combinations thereof.

Notably, the inventors have surprisingly found that basically any change of the physicochemical conditions may result in the destabilization of unloaded PPCs, while loaded PPCs prove to be more stable also to the impact of change of the physicochemical conditions. Thereby, the increased susceptibility of unloaded PPCs versus loaded PPCs to the respective destabilization method can be determined with standard measures known by the person skilled in the art.

It will be understood by the person skilled in the art that in case of pressure filtration and/or pressure chromatography, such as pressure SEC, the method can be applied in step a) to destabilize the unloaded PPCs, and subsequently be applied with same or changed conditions, in particular without applying pressure, as step b2) of separating.

In an embodiment of the present invention changing the physicochemical conditions comprises the change of at least two or more physiochemical conditions at the same time. For example, the change of temperature and pressure at the same time may result in an advantageous specific destabilization of unloaded PPCs. Particularly, the at least partially simultaneous increase of temperature, more particularly heat denaturation, and the decrease of pressure, or the at least partially simultaneous increase of temperature, more particularly heat denaturation, and the increase of pressure may be advantageously applied during step a) of subjecting the mixture to destabilization according to the present invention.

In a still further preferred embodiment of the method according to the present invention the protease is selected from the group comprising exopeptidases and endopeptidases, preferably the protease is selected from the group comprising aminopeptidase, dipeptidase, dipeptidyl-peptidase, peptidyl-dipeptidase, serin-carboxypeptidase, metallo-carboxypeptidase, cystein-carboypeptidase, omega-peptidase, serin-endopeptidase, cysteine-endopeptidase, aspartate-endopeptidase, metallo-endopeptidase, and threonine-endopeptidase, more preferably the protease is a serin-endopeptidase, most preferably the serin-endopeptidase is trypsin.

Particularly, the protease can be also selected from the proteases depicted in below Table 1.

**Table 1: Alternative proteases**

| Protease | Cleavage Site | Optimal pH |
|---|---|---|
| **Lys-C and rLys-C** | NNNKvNNN (K is lysine) | 7.0-9.0 |
| Specific Protease | | |
| **Arg-C** | NNNNRvNNN (R is arginine) Arg-C can, at a lesser degree, cleave at lysine also. | 7.6 - 7.9 |
| Specific Protease | | |
| **Glu-C** | NNNN**E**vNNN (**E** is glutamate) Glu-C can, at a lesser degree, cleave at aspartate residue also. | 4.0-9.0 |
| Specific Protease | | |
| **Asp-N and rAsp-N** | NNNNv**D**NNN (**D** is aspartate) | 4.0-9.0 |
| Specific Protease | | |
| **Chymotrypsin** | NNNN(**F/Y/W**)vNNN (**F,**Y and **W** are aromatic residues phenylalanine, tyrosine and tryptophan) | 7.0-9.0 |
| Low Specific Protease | | |
| **Pepsin** | Nonspecific Protease (advantage: most active at low pH) | 1.0 - 3.0 |
| Nonspecific Protease | | |
| **Thermolysin** | Nonspecific Protease (advantage: remains active at high temperature) | 5.0-8.5 |
| Nonspecific Protease | | |
| **Elastase** | Nonspecific Protease | 9.0 |
| Nonspecific Protease | | |
| **ProAlanase^{™}** | C-terminal side of proline and alanine amino acids | -1.0-5.5 |

Protease selection preferably depends on the amino acid composition of the PPC.

A person skilled in the art will immediately recognise that the use of a protease, like trypsin, may also advantageously or disadvantageously affect the surface of the PPC. Thereby, particularly the tropism of a PPC, and more particularly of a VLP, may be altered. Thereby the present inventors have understood that such change of surface of the PPC may be advantageously used to alter the surface of the PPC in a desired direction. The person skilled in the art thereby also knows various methods to determine the impact of the protease treatment to the surface of the PPC, and thus is enabled to select both the specific protease and the PPC composition to advantageously and/or not negatively impact the desired effect of the PPC application.

Also the conditions of the treatment with a destabilisation agent, particularly a protease, may advantageously or disadvantageously impact the result of destabilisation. Particularly, the duration of adding a destabilizing agent, concentration of the destabilizing agent or the specific conditions of destabilization, and temperature may influence the desired increased percentage of loaded PPC. The person skilled in the art is aware of the variables that have an impact on destabilization and can adjust the conditions accordingly which are preferably titrated for each combination of PPC and cargo.

A longer treatment duration thereby may be advantageous to increase the destabilization of unloaded PPCs. However, destabilization for a longer time period may also result in disadvantageous effects. Therefore, the optimal duration is chosen depending on the specific protease and the PPC composition, and may most preferably not exceed 1 hour.

In a still further preferred embodiment of the method according to the present invention the protease is added in step a) to the mixture at a final concentration optimal for the destabilization of unloaded PPCs, and preferably in a concentration range of 0 to 1 mg/ml. Proteases are preferably used according to each protease's specificities which are known to the person skilled in the art.

In a still further preferred embodiment of the method according to the present invention the temperature of protease treatment for destabilization may preferably be chosen to be the optimal efficacy temperature of the respective protease.

Particularly, after adding the protease, the mixture thus may be incubated at a temperature range between 16°C and 75°C.

The person skilled in the art knows the optimal temperature of specific enzymes, and particularly nucleases and proteases and may choose a temperature, suitable to provide the desired effect, and thereby, preferably also considers adjusting further influencing factors including time and concentration. Thereby, the person skilled in the art knows various methods to determine optimal conditions applying standard adjustment methods.

In a still further preferred embodiment of the method according to the present invention before and/or after step b), the method additionally comprises a step of adding a nuclease, preferably selected from the group comprising endonuclease and exonuclease, more preferably selected from the group comprising DNAse and RNAse. A nuclease treatment is preferably done if a nucleic acid is used as cargo. Different nucleases are known to the skilled person and include, for example, Benzonase^{®} which is both a DNAse and an RNAse.

It is to be noted that in an embodiment of the present invention the destabilization agent may be a nuclease. This may be particularly advantageous, if nucleic acids are associated or attached to the surface of the PPC. Also here the present inventors have surprisingly found that the unloaded particles may be less stable to such nuclease treatment compared with loaded PPCs. This step may particularly be useful and included in the inventive method, if no further nuclease step is present in the protocol before. Otherwise, such associated or attached surface nucleic acid may have been already digested.

The person skilled in the art knows at what conditions the nucleases and/or proteases shall be used.

In a still further preferred embodiment of the method according to the present invention a nuclease is added in a step a) of subjecting the mixture to destabilization as a destabilization agent, in addition to a destabilization agent, particularly a protease.

In a still further preferred embodiment of the method according to the present invention a nuclease is added subsequent to a step a) of subjecting the mixture to destabilization.

In a still further preferred embodiment of the method according to the present invention the nuclease is a DNAse, in cases where RNA, particularly mRNA, is selected as the cargo molecule; and/or the nuclease is an RNAse, in cases where DNA is selected as the cargo molecule and/or the nuclease is both DNAse and RNAse (such as for example a Benzonase^{®}).

The person skilled in the art also knows that depending on the choice of nuclease, buffer and salt concentrations in the reaction mixture have to be adjusted according to methods known in the art. This particularly allows to control reaction parameters, and particularly the nuclease activity, via an adjustment of buffer composition.

A nuclease treatment according to the present invention may particularly be carried out at a temperature range of about 16°C to about 60°C, and/or at a pH of about 1 to 9.

In a still further preferred embodiment of the method according to the present invention the protein- or peptide-based capsule comprises a capsid protein derived from a virus selected from the group comprising Parvoviridae, particularly adeno-associated virus; Retroviridae, particularly Lentivirus, such as HIV; Flaviviridae, particularly Hepatitis C virus; Hepadnaviridae, particularly Hepatitis B virus; Paramyxoviridae, particularly Nipah or measles virus; Polyomaviridae, particularly John Cunningham virus (JCV); Togaviridae, particularly Sindbis virus; Reoviridae, particularly rotavirus; Riboviridae, particularly Respiratory syncytial virus Picornaviridae, particularly foot-and-mouth-disease virus; Papillomaviridae, particularly human Papilloma virus; Caliciviridae, particularly Norwalk virus; Nodaviridae, particularly Flock House Virus; Virgaviridae, particularly Tobacco mosaic virus; bacteriophages, particularly Qβ, or AP205; and RNA phages.

It shall be understood that the method according to the present is in essence applicable to any kind of PPC, and particularly VLP, depending on the particular composition of the capsule.

In an embodiment of the present invention the PPC is present in the form of an AAV-particle. Thereby, the PPC capsule comprises a capsid protein derived from adeno-associated virus.

Adeno-associated viruses (AAV) belong to the family of Parvoviridae and are small viruses that infect humans and some other primate species. AAV are usually known to not cause disease and merely induce a mild immune response. This lack of pathogenicity makes AAV particles which comprise AAV capsid proteins in the capsule an attractive carrier for various applications conveying in vivo expression, and particularly a preferred vehicle for gene therapies. Particularly AVVs have linear single-stranded DNA (ssDNA) genome of about 4.5 kb, which is usually flanked by two inverted terminal repeats (ITR) and two open frames, rep and cap. Rep gene encodes regulatory proteins involved in genome replication (Rep 78/68) and packaging (Rep 52/408). Cap gene encodes 3 capsid proteins: VP1, VP2 and VP3 in a ratio of 10:1:1. Thereby, cap defines the AAV's cell tropism. The person skilled in the art knows the selective targeting of different cell types and tissues that can be advantageously achieved using AVVs and one of its various serotypes which express different tissue tropism. Based on this feature, certain serotypes can be chosen to target a specific tissue or cell type. The person skilled in the art further knows to apply modifications of promoter or capsid to allow generating AAV which target specific tissues or cells.

In another embodiment, PPC derived from Lentivirus, for example Lentivirus capsids, may be used, for example from HIV.

In a still further preferred embodiment of the method according to the present invention the protein- or peptide-based capsule is present in the form of a virus-like particle (VLP).

In a still further preferred embodiment of the method according to the present invention the protein- or peptide-based capsule is capable of passing intact physiological and biological barriers, preferably blood brain barrier (BBB), in vivo.

The term physiological barrier as used herein, preferably refers to any barrier of the physiological system, and particularly single layers of endothelial cells, particularly non-fenestrated endothelial cells. The capability of the PPCs of passing of such intact physiological barriers may thus be advantageous to allow the cargo molecule to be delivered to its target site.

In a still further preferred embodiment of the method according to the present invention the cargo molecule comprises or is an RNA, a DNA, a protein or a combination thereof, preferably an mRNA and/or an antibody. Such cargo molecule may particularly exploit its coded function, or effect of its transcript at the target site, respectively.

In a still further preferred embodiment of the method according to the present invention the cargo molecule is selected form the group consisting of a protein, a peptide or a nucleic acid, in particular selected from the group consisting of nucleic acids encoding a desired protein such as mRNA, cDNA, a plasmid or vector, inhibitory nucleic acids such as siRNA or miRNA, particularly short RNA selected from siRNA, shRNA, oRNA, sshRNA, IshRNA, ASO and miRNA, and nucleic acids having catalytic activity such as a ribozyme. In a further embodiment, the cargo molecule may also be selected from hybrid molecules of the above-named molecules, and particularly a hybrid molecule of a first nucleic acid and second nucleic acid, such as RNA/DNA or a hybrid molecule of a nucleic acid and a peptide or protein, such as a DNA/Protein, or DNA/Peptide hybrid molecule - any other combination are also considered herein.

It is particularly preferred to provide a gene, such as in form of cDNA or mRNA, as cargo in order to provide a DDS comprising such PPC with such cargo in a gene therapeutic approach. A gene therapeutic approach may be useful to replace a defective gene, in particular in a disease, such as LSDs, Canavan disease or Krabbe disease.

Particularly, where the cargo molecule is a protein, the cargo protein may be provided in the form of a growth factor.

In a still further preferred embodiment of the method according to the present invention the cargo molecule comprises or is a dye, a diagnostic agent and/or an inorganic compound. In embodiments, wherein the cargo molecule comprises or is a dye may allow advantageously the use of the PPCs in methods of molecular imaging. The inorganic compound may be comprised in the cargo molecule as a molecule as such or as a moiety. Examples include contrast agents, metalls, diagnostic markers, tumor markers, K, Ca, Mg and the like.

In a still further preferred embodiment of the method according to the present invention the protein- or peptide-based capsule fully incorporates the cargo molecule.

In a still further preferred embodiment of the method according to the present invention the cargo molecule may particularly be a pharmaceutically acceptable product, also referred to herein as drug, particularly an active pharmaceutical ingredient (API), a biological or a cytostatic.

The term "pharmaceutically acceptable product" as used herein, preferably refers to a therapeutic or diagnostic substance. It can be of any chemical nature as long as it may be loaded in and/or associates with the PPC. Preferably the pharmaceutically acceptable product is an active pharmaceutical ingredient (API), in particular a "small molecule" (preferably an organic compound of ≤ 1000 Daltons), a biological or a cytostatic. In a preferred embodiment the biological is selected form the group consisting of a protein, a peptide or a nucleic acid, in particular selected from the group consisting of nucleic acids encoding a desired protein such as mRNA, cDNA, a plasmid or vector, inhibitory nucleic acids such as siRNA or miRNA and nucleic acids having catalytic activity such as a ribozyme.

In a still further preferred embodiment of the method according to the present invention the cargo molecule is selected from the group consisting of antibodies, antipsychotic drugs, analgesic drugs, thrombolytics, antidepressants, immunomodulators, immunosuppressants, acetylcholinesterase inhibitors, glutamate receptor antagonists or modulators, such as NMDA receptor antagonists, psychostimulants, anti-dementia drugs, anxiolytic drugs, nootropic drugs, metabolic enhancers, metabolic modulators, neuroprotective drugs, anticonvulsants, cytostatic, and cytokines.

In a still further preferred embodiment of the method according to the present invention the cargo molecule is a pharmaceutically acceptable product, particularly an active pharmaceutical ingredient (API). The term "API" preferably implies that the API is suitable in a method of treatment and/or diagnosis, preferably for the treatment and/or diagnosis of neurological disorders, in particular CNS diseases.

In a further aspect of the present invention the invention relates to a composition comprising protein- or peptide-based capsules loaded with a cargo molecule obtainable by the method according to any of the embodiments of the inventive method. The cargo molecule may in particular be an RNA, a DNA, a protein, or a combination thereof, a dye, a diagnostic agent and/or an inorganic compound.

This composition allows handling the PPC under physiological conditions. Under these conditions the PPCs essentially remain intact, preferably they essentially maintain their capsule structure. If loaded with cargo, they essentially remain associated with the cargo.

In a still further preferred embodiment of the method according to the present invention the composition is for use in a method of *in vivo* diagnosis of a disease or disorder, preferably for the *in vivo* diagnosis of neurological disorders, in particular of CNS diseases, in a subject in need thereof.

For example, the PPC may be loaded with a dye, such as a contrast agent, and injected into a subject for the molecular imaging of specific structures in the body, for example in the CNS. The visualization of specific structures allows a diagnosis.

Hence, in a still further preferred embodiment of the method according to the present invention the composition according to the present invention is for use in a method of molecular imaging, particularly the cargo molecule being a dye or another diagnostic agent and the method more particularly being for use in a method of *in vivo* diagnosis.

In a further aspect of the present invention the invention relates to the use of the composition according to the present invention for molecular imaging.

In a still further aspect of the present invention the invention relates to the use of the composition according to the present invention in a method of *in vivo* diagnosis. Accordingly, the invention also relates to the use of the composition according to the present invention in the manufacture of an *in vivo* diagnostic.

In a further aspect of the present invention the invention relates to the use of a composition as described herein in a method of *in vivo* diagnosis of a disease or disorder, preferably for the diagnosis of neurological disorders, in particular of CNS diseases, in a subject in need thereof.

In a still further preferred embodiment of the method according to the present invention the composition as described herein in accordance of the present invention is for use in a method of diagnosis, wherein the protein- or peptide-based capsules are capable of passing intact physiological and biological barriers, particularly the blood brain barrier (BBB), particularly the physiologically intact BBB in vivo, and wherein more preferably the crossing does not require a loss of integrity or increased permeability of the BBB.

In a further aspect of the present invention the invention relates to the use of a composition as described herein in a method of *in vivo* diagnosis of a disease or disorder, wherein the protein- or peptide-based capsules are capable of passing intact physiological and biological barriers, particularly the blood brain barrier (BBB), particularly the physiologically intact BBB in vivo, and wherein more preferably the crossing does not require a loss of integrity or increased permeability of the BBB.

Preferably, the method according to the present invention does not require a loss of integrity or increased permeability of the BBB.

The integrity of the BBB in vitro may be measured by known methods, for example by relative trans endothelial electrical resistance measurement (TEER) (Rempe et al., Biochem Bioph Res Comm 2011, 406 (1): 64-69). Many in vitro models of BBB are established, including primary bovine or human brain endothelial cells in different co-cultures, for example the human brain endothelial cell line HBEC-5i. In vivo, imaging methods, such as CT scans or MRI, can be used together with contrast agents to visualize BBB permeability. Functional imaging, such as PET or SPECT, may also be used.

The composition comprising the drug delivery system preferably does not require an additive that may disrupt the integrity of the BBB. Hence, in a most preferred embodiment of the invention the drug delivery system is free of any additive that can impact the permeability of the BBB.

In a still further aspect of the present invention the invention relates to a Drug Delivery System (DDS) obtainable by a method according to the present invention, and particularly a method according to the first aspect of the invention.

In the context of the invention, the term "drug delivery system" refers to a composition for administering a pharmaceutically acceptable product to a subject in need thereof, in particular to a human or animal. A drug delivery system, advantageously, enables the delivery of the pharmaceutically acceptable product contained therein or attached thereto to a site of interest, preferably in a human or animal. Preferably the delivery is selective for the target, i.e. more of the pharmaceutically acceptable product is delivered to the target than to other sites of the body or organ.

In another aspect, the invention relates to a drug delivery system obtainable by the method according to the invention. Such a drug delivery system has the advantages as stated supra. In particular, such a drug delivery system can be used in a method of treatment and/or diagnosis, preferably for the treatment of neurological disorders, i.e. CNS diseases. The drug delivery system can be used in a method of treatment and/or diagnosis, preferably for the treatment of neurological disorders. Hence the invention also relates to a method of treatment a disorder, in particular a CNS disease, with the drug delivery system according to the invention. The method of treatment preferably comprises the step of administering the drug delivery system to a subject in need thereof.

Hence the invention also relates to a method of treating a disorder, in particular a CNS disease, with the drug delivery system according to the invention. The method of treating preferably comprises the step of administering the drug delivery to a subject in need thereof. Accordingly, the invention also relates to the use of a drug delivery system according to the present invention in the manufacture of a medicament for the treatment and/or *in vivo* diagnosis of a disease or disorder.

The invention also relates to the use of the drug delivery system for the manufacture of a medicament for the treatment of neurological disorders, i.e. CNS diseases. The method of treatment preferably does not comprise a step of increasing the permeability of the BBB of the subject to be treated. The drug delivery system of the invention, preferably, is administered to a patient who has not received any chemical or physical treatment for impairing or disrupting the BBB.

The term "CNS diseases" (or "neurological disorders") refers to any disorder of an individual's nervous system, preferably of the central nervous system. It encompasses neurodegenerative, psychotic or neurovascular disorders.

In one embodiment of the invention, the neurological disorder is selected from the group consisting of stroke, alcohol addiction, Alzheimer Disease, anxiety, arthritis, asthenia, attention deficit hyperactivity disorder, bipolar disorder, cancer pain, cerebral ischemia, cerebral neuroprotectant, cervical dystonia, Chorea associated with Huntington's disease, chronic pain, chronic severe pain, cognitive disorder, cortical myoclonus, degenerative Nerve Diseases, depression, diabetic neuropathic pain, diabetic neuropathy, emotional lability, epilepsy, excessive sleepiness associated with narcolepsy, fibromyalgia, Fragile X syndrome, Friedreich's ataxia, insomnia, Lennox Gastaut syndrome, major depressive and anxiety disorders, manic episodes associated with bipolar disorder, memory impairment, migraine, mild cognitive impairment, moderate to severe pain, motor neuron disease, multiple sclerosis, musculoskeletal pain, narcolepsy, neuralgia, neuropathic pain, nicotine dependence, obsessive compulsive disorder, opioid-induced adverse effect, opioid-induced constipation, osteoarthritis pain, overactive bladder, pain, Parkinson's disease, pediatric drooling, peripheral diabetic neuropathy, post-operative pain, postherpetic neuralgia, premenstrual dysphoric disorder, psychosis, refractory complex partial seizures, restless leg syndrome (RLS), schizophrenia, seizure, severe chronic pain, sleep disorder, smoking cessation, spasticity, spinal cord injury, stroke, transthyretin familial amyloid polyneuropathy, traumatic brain injury, vertigo, cachexia, amyotrophic lateral sclerosis, spinocerebellar ataxia type I, extrapyramidal and movement disorders, transient ischemic attack (TIA), Progressive multifocal leukoencephalopathy (PML), HIV-infection, dementia, such as Alzheimer's disease, vascular dementia, frontotemporal dementia, semantic dementia and dementia with Lewy bodies, and preferably selected from the group comprising or consisting of Alzheimer's disease, Parkinson's disease and multiple sclerosis.

In a particularly preferred embodiment of the invention, the DDS is used in a method of treating Lysosomal storage diseases (LSD), Canavan disease and/or Krabbe disease.

LSDs are a group of about 50 inherited diseases. LSDs mostly involve the dysfunction of lysosomal hydrolases, which result in impaired substrate degradation. As a consequence, incompletely processed cellular material accumulates within various tissue types, and disease-specific clinical manifestations occur.

Beyond substrate degradation, lysosomes are involved in energy homeostasis, generation of building blocks for cell growth, mitogenic signaling, the priming of tissues for angiogenesis and metastasis formation and activation of transcriptional programs.

One example of a lysosomal storage disease is the metachromatic leukodystrophy (MLD). MLD is characterized by a diminished activity of arylsulfatase A (ASA) caused by mutations in the ASA gene affecting the metabolism of sphingolipids. Without ASA, sulfatides build up in many tissues of the body, eventually destroying the myelin sheath of the nervous system. Symptoms involve developmental delays, weakness, muscle rigidity, mental deterioration, dementia and blindness. Hence, a gene therapeutic approach providing the ASA gene would be beneficial.

Canavan disease and Krabbe disease are examples of leukodystrophies. Leukodystrophies are a group of rare, progressive, metabolic, genetic diseases that affect the brain, spinal cord and often the peripheral nerves. Each type of leukodystrophy is caused by a specific gene abnormality that leads to abnormal development or destruction of the white matter (myelin sheath) of the brain. Each type of leukodystrophy affects a different part of the myelin sheath, leading to a range of neurological problems.

One example of a leukodystrophy is Canavan disease (CD). CD is a rare inherited neurological disorder characterized by spongy degeneration of the brain and spinal cord (central nervous system). Physical symptoms that appear in early infancy may include progressive mental decline accompanied by the loss of muscle tone, poor head control, an abnormally large head (macrocephaly), and/or irritability. Physical symptoms appear in early infancy and usually progress rapidly.

CD is caused by a defective ASPA gene which is responsible for the production of the enzyme aspartoacylase. Decreased aspartoacylase activity prevents the normal breakdown of N-acetyl aspartate, wherein the accumulation of N-acetyl aspartate, or lack of its further metabolism interferes with growth of the myelin sheath of the nerve fibres of the brain. Hence, a gene therapeutic approach providing the ASPA gene would be beneficial.

Another example of a leukodystrophy is Krabbe disease. Krabbe disease, also known as globoid cell leukodystrophy, is an autosomal recessive lipid storage disorder caused by mutations in the GALC gene located on chromosome 14 (14q31), which is inherited in an autosomal recessive manner. Mutations in the GALC gene cause a deficiency of the lysosomal enzyme galactocerebrosidase, which is necessary for the breakdown (metabolism) of the sphingolipids galactosylceramide and psychosine (galactosyl-sphingosine). Failure to break down these sphingolipids results in degeneration of the myelin sheath surrounding nerves in the brain (demyelination). Characteristic globoid cells appear in affected areas of the brain. This metabolic disorder is characterized by progressive neurological dysfunction with irritability, developmental regression, abnormal body tone, seizures and peripheral neuropathy. Hence, a gene therapeutic approach providing the GALC gene would be beneficial.

Hence, in a still further preferred embodiment of the method according to the present invention the Drug Delivery System (DDS) according to the present invention is for use in a method of treatment and/or *in vivo* diagnosis, preferably for the treatment of neurological disorders, in particular of CNS diseases, in a subject in need thereof.

In a particular preferred embodiment, the neurological disease, in particular the CNS disease, is an LSD, Canavan disease and/or Krabbe disease.

"A drug delivery system for the CNS" means that the drug delivery system selectively targets the CNS. CNS refers to the spinal cord and the brain, in particular to the brain. The term "brain" includes anatomical parts thereof, such as frontal lobe, parietal lobe, temporal lobe, occipital lobe, and cerebellum.

In a still further preferred embodiment of the drug delivery system according to the present invention the protein- or peptide-based capsules cross an intact physiological and biological barrier, particularly the blood brain barrier (BBB), particularly the physiologically intact BBB in vivo, and wherein more preferably the crossing does not require a loss of integrity or increased permeability of the BBB.

The drug delivery system preferably has an improved efficacy due to an increased percentage of loaded PPCs. The PPC can cross the BBB without a prior increase of the permeability of the BBB. Hence, the drug delivery system of the invention can be used in a method of treatment of a CNS disease, wherein the method does not comprise a step of increasing the permeability of the BBB of the subject to be treated. The drug delivery system of the invention, preferably, is administered to a patient who has not received any chemical or physical treatment for impairing or disrupting the BBB.

Importantly, the crossing of the BBB by the drug delivery system enables the drug delivery system to exhibit is function of targeting specific cell populations within the brain, i.e. deliver a cargo to targeted cells. In the context of the invention said drug delivery system comprises a delivery to and/or into the targeted cells.

In a still further preferred embodiment of the method according to the present invention the Drug Delivery System (DDS), preferably comprising a VLP loaded with an mRNA or antibody, is for use in a method of treatment of a disease or disorder, preferably a neurological disorder, in particular a CNS disease.

In a still further aspect of the present invention the invention relates to a method of treatment of neurological disorders, in particular of CNS diseases, in a subject in need thereof, comprising a step of application of the Drug Delivery System (DDS) obtainable by the inventive method to a subject in need thereof.

In a still further aspect of the present invention the invention relates to a method of *in vivo* diagnosis of neurological disorders, in particular of CNS diseases, in a subject in need thereof, comprising a step of application of the composition obtainable by the inventive method to a subject in need thereof.

The embodiments in the figures may relate to preferred embodiments, while all elements and features described in connection with embodiments may be used, as far as appropriate, in combination with any other embodiment and feature as discussed herein, in particular related to any other embodiment discussed further above.

The features of the present invention disclosed in the specification, the claims, examples and/or the figures may both separately and in any combination thereof be material for realizing the invention in various forms thereof.

### MATERIALS AND METHODS

### Treatment of VLPs derived from JCV

### Trypsin

After manufacture of VLPs, trypsin (Sequencing Grade Modified, Promega) with a final concentration of 0.025 µg/µl was added to reassociated VLP and incubated for at least 15 min at 37 °C. After that, samples were placed on ice for at least 5 min prior sterile filtration (0.2 µm). Afterwards final samples were either used directly or shock frozen in liquid nitrogen and stored at -80°C.

### SEC

After trypsinization sample was injected with help of direct injection pump into NGC Chromatography system (BioRad). Size-exclusion chromatography was prepared with help of HiPrep^{™} Sephacryl^{®} S-500 HR (Sigma Aldrich) using 10 mM Tris-HCI, 150 mM NaCl, 2 mM CaCl₂, pH 7.5 buffer. VLP Peak was detected with retention time at 0.5CV. VLPs were collected and concentrated using Vivaspin^{®} centrifugal concentrator with 50 kDa cut-off. Afterwards final samples were characterized and stored at -80°C.

### VLP characterization

For verifying sample stability, inflection temperatures for each sample were assessed by nDSF using a Tycho NT.6 (Nanotemper) according to manufacturer's instruction.

### DLS

For DLS measurement, samples were sterile filtered (0.2µm) and at least 40µl were measured in 10 runs using appropriate cuvettes (UV-cuvettes, Brand).

### RiboGreen^{™} Assay

For quantifying encapsulated cargo amount, RiboGreen^{™} Assay was performed in a 96-well plate according to the manufacturer's instructions (Thermo Fisher Scientific). Fluorescence was measured at 480/520 nm. Encapsulated amount was calculated using calibration curve of the cargo alone.

### qPCR

For determining loading efficiency via qPCR 1µg of pure mRNA matching the mRNA cargo was used in cDNA synthesis. The subsequent cDNA mix was diluted in steps of 10 from 1:500 to 1:50.000.000 and a qPCR was performed for each dilution. The resulting Cq values were plotted against the mRNA quantities in ng, assuming the Cq value of the diluted cDNA is equivalent to the same mRNA dilution. An exponential fit of the standard curve was used to calculate mRNA packaging from Cq values of loaded VLPs.

### Agarose gel electrophoresis (AGE)

For analyzing presence of VLP and verifying co-localization of protein and encapsulated cargo, agarose gel electrophoresis (AGE) was performed using 1% agarose gel and Tris-acetate buffer. Loading buffer was prepared using glycerol and bromphenol blue. Encapsulated cargo was visualized after incubating the gel with GelRed^{™}. To visualize VLPs, gels were incubated with Instant Blue. Images were taken using Biorad Gel Doc^{™} XR+ Gel Documentation System.

**Transmission electron microscopy (TEM)** analyses were carried out for directly visualizing the sample at different experimental steps with help of a Zeiss EM900 electron microscope, operating at a voltage at 80 kV. For this approach, samples were stained on carbon-coated copper grids (Plano GmbH) using 2 % uranyl acetate (Sigma Aldrich) beforehand.

### Flow Cytometry for analyzing attachment of antibodies VLP are loaded with to cells

Glioblastoma cells were seeded 24 h in advance with a density of 3*10⁴ in 900 µl DMEM high glucose medium (GutaMAX^{™}, Thermo Fisher) supplemented with 10 % FCS (Thermo Fisher) and 1 % Pen-Strep (PAN-Biotech). Prior to adding the sample, the corresponding amount of media was removed for keeping a constant amount of medium in each well. VLPs loaded with cy5-labled antibody were added to the wells and incubated at 37°C and 5 % CO₂. After 16 h to 18 h, medium was carefully discarded, and cells were washed two times with PBS. Harvesting of cells was performed using 150 µl Trypsin/EDTA per well. After detachment of cells, cells were carefully resuspended using 0.05 % FCS in 500 µl PBS. For flow cytometric investigations, cells of two wells were pooled and centrifuged at 500 x g for 5 min. The supernatant was discarded, and cells were resuspended in approx. 200 µl PBS. Cells were directly used for flow cytometry.

### Artificial blood-brain-barrier (BBB) model for verifying BBB permeation

Fresh inserts were placed in a 24-well plate filled 900 µl DMEM-F12 medium (Thermo Fisher) supplemented with 10 % FCS (Thermo Fisher) and 1 % Pen-Strep (PAN-Biotech). Afterwards, HBEC-5i cells were seeded into inserts using 7.5*10⁴ in 200 µl medium and incubated for at least 5 days (37°C, 5% CO₂). Medium was changed in appropriate intervals.

At the day of the start of the experiment, inserts were transferred to a 24-well plate, containing target cells (glioblastoma cells), that were seeded 24 h in advance with a density of 3*10⁴ in 900 µl DMEM, high glucose, GutaMAX^{™} (Thermo Fisher) supplemented with 10 % FCS (Thermo Fisher) and 1 % Pen-Strep (PAN-Biotech). Prior to adding the sample (VLP packed with Luciferase mRNA) to the insert, the corresponding amount of media was removed for keeping a constant amount of 200 µl medium in each insert. After 48 h, the inserts were placed in a fresh 24-well plate and washed using PBS. Target-cell containing 24-well plate was washed in a comparable manner. Accordingly, luciferase activity was determined according to the manufacturer's instructions (Luciferase Assay, Promega).

### VP1 ELISA

*Sample Lysis.* One thawed mouse brain hemisphere (ca. 215-240 mg) per animal was lysed using the GentleMACS^{™} Dissociator (Miltenyi Biotec). The hemisphere was added into GentleMACS^{™} M Tube with 1 ml TBS-T per 100 mg of tissue and lysis performed using the Protein_01_01 program. Tubes were put on ice for 30 min before centrifugation at 3000xg for 10 min at 4°C and supernatant and pellet were separately snap-frozen with N2 and stored at -80°C and used for subsequent protein analysis.

*MSD VP1 Immunoassay* - *Plate.* MSD 96-well plates (Meso Quick Plex Standard) were coated with 100 µl PBS per well, and, after removal of liquid, incubated with 30 µl of anti-VP1 antibody overnight at 4°C. Plates were washed four times with PBS-T and blocked with 5% milk in PBS-T for 1h at RT, 800 rpm and washed again four times with PBS-T just before addition of samples & standards.

*MSD VP1 Immunoassay* - *Samples & VP1 standards.* Brain supernatant (SN) and pellet lysates were diluted 1:1 with TBS-T and supplemented with DTT (5 mM final) and EDTA (10 mM final). VP1 Standards were spiked into equivalent lysates (brain, brain pellet) of buffer control animals. Samples & Standards were incubated for 30 min at room temperature, 450 rpm shaking. 25 µl of samples & standards were added onto plates and incubated for 2h at room temperature. Plates were washed four times with PBS-T and 25 µl of 4 ng/µl anti-VP1 antibody were added to each well and incubated for 1h at room temperature. Plates were washed four times with PBS-T and incubated with 25 µl of 0.5 ng/µl goat anti rabbit Sulfo-Tag antibody for 1h at room temperature. Plates were washed four times with PBS-T and 150 µl MSD Read Buffer A per well was added. Readout was performed with MESO QuickPlex SQ 120MM reader. Standard curves were fitted with a 4-PL fit with 1/Y^2 weighting and used to calculate VP1 sample concentrations (Graphpad Prism). Amounts of VP1 per organ were calculated considering lysate dilution and organ weight and relative amounts of supernatant and pellet.

### Luciferase expression in vivo: animal study, RNA Isolation, cDNA Synthesis and qPCR

SKH1 mice (2-3 per group) were injected with VLP into tail vein, imaged for bioluminescence (BLI) and terminated 24h after injection. BLI was prepared with help of Night Owl II, LB983NC100 (Berthold Technologies) device and software: IndiGo, Version 2.0.5.0 (Berthold Technologies). 5 ml/kg D-Luciferin (30 mg/ml in PBS; Biosynth, L-8220) were injected intraperitoneally 10 - 15 min before bioluminescence imaging (BLI) to each mouse. The whole mouse was imaged at ventral and dorsal position for 5 minutes. The BLI signals were quantified in counts per second (cps) using the software IndiGo (Version 2.0.5.0) using a defined detection area. The areas of interest were placed around the head and defined as region of interest (ROI).

After termination mice were perfused with PBS and brains were taken out and shock frozen.

One thawed mouse brain hemisphere per animal (n=2-3 per group) was lysed with RA1-Buffer from the NucleoSpin^{®} RNA Isolation Kit (Macherey-Nagel) using the GentleMACS^{™} Dissociator (Miltenyi Biotec) and RNA isolation was performed according to the instructions of the NucleoSpin^{®} RNA Isolation Kit (Macherey-Nagel). RNA concentration was determined using NanoDrop. For cDNA synthesis 1000 ng RNA were used. cDNA Synthesis was performed using the RevertAid^{™} First strand cDNA synthesis kit (Thermo Fisher Scientific) according to manufacturer's instruction. qPCR was prepared using 10µl SsoFast^{™} EvaGreen^{®} Supermix (Biorad), 2 µl Luciferase forward and reverse primer, 1 µl H2O and 5 µl of a 1:5 cDNA dilution. For the qPCR Biorad Opus 384 Thermal Cycler was used and fold-change in comparison to buffer control was calculated.

### EXAMPLES

### Example 1: Examples of Possible Use of Inventive Method

The method for purification of PPCs loaded with a cargo molecule according to the present invention can be advantageously used to increase the percentage of loaded PPCs in a mixture comprising said PPCs loaded with a cargo molecule and unloaded PPCs. Thereby, the method comprises at least the step a) of subjecting the mixture to destabilization. In said step a) a destabilizing agent can be added, and additionally or alternatively the physicochemical conditions can be changed. Thereby, the unloaded PPCs are preferably destabilized compared to the loaded PPCs, and thus the percentage of loaded PPCs is advantageously increased in the mixture. Optionally but preferably, in a further, and more preferably subsequent step b1) the mixture is filtrated. This can advantageously remove non-capsule material, particularly aggregates. Still optionally but preferably, and in a still further step b2), preferably carried out subsequent to step a) and/or step b1) the mixture is subjected to a step b2) of separating. This step advantageously results in a removal of unloaded PPCs from the mixture. As a result the percentage of loaded PPCs in the mixture is advantageously increased and the mixture is more advantageously purified for loaded PPCs.

The person skilled in the art will thus immediately recognize that the method according to the invention has broad and advantageous applicability in the art of PPC manufacturing, particularly, in the manufacturing of Drug Delivery Systems and compositions of other use, such as in vivo live imaging, comprising PPCs as carrier of cargo molecules.

Such methods are for example disclosed in WO 2020/125962, wherein a method for providing a drug delivery system comprising a virus-like particle (VLP) derived from John Cunningham virus (JCV) is described.

As shown schematically in Figure 1 the method for purification of PPCs loaded with a cargo molecule according to the present invention and thus the enrichment of loaded PPCs can be conducted after a manufacturing process of providing such drug delivery systems as cited above.

### Example 2: Stability, Purity, and Packaging verified by agarose gel electrophoresis

In Figure 2 an Agarose Gel Electrophoresis (AGE) analysis is shown. In this example the presence of PPCs is verified by co-localization of protein and encapsulated mRNA cargo molecules. The gel photography shown on the left is detected in the fluorescence channel after GelRed^{®} stain, and thus shows the fluorescenting mRNA molecules in the samples. The gel photography shown on the right is detected in brightfield after InstantBlue^{®} protein stain, and thus shows the protein load in the samples. From left to right the samples loaded to the gel refer to L) Ladder; 1) Bulk before trypsin; 2) Bulk after trypsin; 3) Purified after trypsin (SEC); 4) mRNA control.

VLP were further treated with trypsin or with trypsin and subjected to SEC. It can thus be seen that trypsin digestion and further SEC purification showed a single VLP fraction with high mRNA association. Also, tiny particles (present in 1) visible as the lower band) were advantageously reduced by trypsin treatment, whereas loaded VLPs (PPCs) were found to be stable towards trypsin digestion.

### Example 3: increase of mRNA cargo molecule per PPC

In Example 3, the increase of mRNA cargo molecules per PPC has been determined after inventive trypsin destabilization and subsequent SEC purification. As can be seen from Figure 3 the quantification of the amount of encapsulated mRNA cargo molecules with a RiboGreen^{™} Assay after trypsin digestion without and with subsequent SEC treatment results in an about 2-fold increase of mRNA per PPC amount. Approximately 2 mRNA cargo molecules per PPC were calculated using RiboGreen.

For verifying sample stability, inflection temperatures (Ti) for each sample were assessed by nDSF using a Tycho NT.6 (Nanotemper).

The results for different representative test samples are depicted in Table 2 below.

The different test samples are chosen to comprise different polyA tails and modifications. Each sample was subjected to the method according to the present invention comprising a destabilization step a) with trypsin treatment and a subsequent step b2) of separation applying SEC. As can be seen from Table 2, each sample was tested after step a) (After Trypsin) and after step b2) (After SEC).

It can be taken from the Ti values of Table 2 that even samples with high Ti can be improved by Trypsin-SEC treatment.

**Table 2: Inflection temperatures (Ti) of PPCs loaded with different mRNAs**

| **Sample** | **After Trypsin** | **After SEC** |
|---|---|---|
| VLP loaded with mRNA1 | 74.3 | 75.5 |
| VLP loaded with mRNA2 | 74.3 | 74.9 |
| VLP loaded with mRNA3 | 74.1 | 74.4 |
| VLP loaded with mRNA4 | 73.4 | 74.8 |

### Example 4: Quantification of PPCs loaded with mRNA with a RiboGreen^{™} Assay

In Example 4 the amount of mRNA cargo molecules per PPC was determined for two further PPC sample mixtures (loaded with cargo molecules mRNA1 and mRNA2) purified with the method according to the present invention. For this purpose, a RiboGreen^{™} Assay was performed after subsequent application of step a) of subjecting the mixture to destabilization by adding trypsin as a destabilizing agent and further subsequent step b2) of separation by application of SEC treatment in accordance to the method of the present invention.

As shown in Figure 4 also in this example the ratio mRNA molecule per PPC can be advantageously increased by applying step b2) of separating with SEC compared to samples that were not subjected to step b2) of separating with SEC according to the present invention.

As can be immediately seen from Figure 4, after SEC treatment, more than 80% of PPCs are loaded with one mRNA cargo molecule. Particularly a significant increase of mRNA per PPC ratio could be detected for both samples of mRNA1 and mRNA2 after trypsin destabilization and SEC separation. It can be immediately seen that the ratio of cargo molecules per PPC as calculated using RiboGreen could in both samples, mRNA1 and mRNA2, be increased from about 1 mRNA per PPC to about 2 mRNA per PPC.

Further, an alternative method to a RiboGreen^{™} assay is used (pPCR). The method also allows to quantify mRNA particles per PPC. Figure 5 shows two different samples after trypsin and SEC treatment which reveal 1.2605 and 0.8530 mRNA per PPC.

### Example 5: Quantification of PPCs loaded with mRNA via Transmission electron microscopy (TEM)

Figure 6 shows TEM images of a mixture comprising PPCs loaded with mRNA molecule as a cargo molecule and unloaded PPCs after a trypsin destabilization step and with and without subsequent application of a step b2) of SEC separation according to the present invention. As can be readily seen by comparison of the left TEM picture (after trypsin without SEC) and the right TEM picture (After trypsin with SEC) stability and homogeneity of PPCs loaded with mRNA as cargo molecule can be proven after treatment. It is readily visible that in the sample shown on the left, i.e. before performing the step b2) of separation according to the method of the present invention, the sample contains loaded PPCs, but also tiny particles and pentamers (as well as mRNA-VP1 associates). It can be concluded that due to trypsin treatment the sample on the left comprises destabilized PPC.

### Example 6: Quantification of PPCs with plasmid molecule (pNL) as cargo molecules via Transmission electron microscopy (TEM)

In Example 6 plasmid pNL (3.9kb) was used as cargo molecule and loaded into PPCs.

TEM was performed to analyze sample stability and purification. Figure 7 shows TEM images of a mixture comprising PPCs loaded with plasmid molecule (pNL) as a cargo molecule and unloaded PPCs after a trypsin destabilization step and with and without subsequent application of a step b2) of SEC separation according to the present invention. As can be readily seen by comparison of the left TEM picture (after trypsin without SEC) and the right TEM picture (After trypsin with SEC) stability and homogeneity of PPCs loaded with a plasmid as cargo molecule can be proven after treatment. It is readily visible that in the sample shown on the left, i.e. before performing the step b2) of separation according to the method of the present invention, the sample contains loaded PPCs, but also tiny particles and pentamers.

### Example 7: Quantification of PPCs with plasmid molecule (pGL4.51) as cargo molecules via Transmission electron microscopy (TEM)

In Example 7 plasmid pGL4.51 (6.4kb) that varies significantly in size from the plasmid molecule pNL (3.9kb) as used in Example 6 was used as cargo molecule and loaded into PPCs.

TEM was performed to analyze sample stability and purification. Figure 8 shows TEM images of a mixture comprising PPCs loaded with plasmid molecule (pGL4.51) as a cargo molecule and unloaded PPCs after a trypsin destabilization step and with and without subsequent application of a step b2) of SEC separation according to the present invention. As can be readily seen by comparison of the left TEM picture (after trypsin without SEC) and the right TEM picture (After trypsin with SEC) stability and homogeneity of PPCs loaded with a larger plasmid as cargo molecule can be proven after treatment. It is readily visible that in the sample shown on the left, i.e. before performing the step b2) of separation according to the method of the present invention, the sample contains loaded PPCs, but also tiny particles and pentamers (as well as mRNA-VP1 associates)

Accordingly, from Example 6 and 7 it can be readily seen that plasmids of different size can be used as cargo molecules to load PPCs and advantageously treated with the method for purification according to the present invention. Trypsin treatment leads to destabilization of unloaded PPCs.

### Example 8: Luciferase activity measured in glioblastoma cells transfected with VLP loaded with Luciferase mRNA

In Example 8 Luciferase activity was measured in glioblastoma cells transfected with VLP loaded with Luciferase mRNA in apical or basolateral subcellular localization. The result is shown in Figure 9.

Example 8 was conducted with the aim to check, if a method of purification according to the present invention, and particularly a step a) of destabilization with trypsin as a destabilization agent reduced permeation by digesting surface protein structures.

For this reason an artificial blood-brain-barrier (BBB) model for verifying BBB permeation was used. As can be readily seen from the measured luciferase signals the impact of agglomerates/tinies was reduced by using the method for purification according to the present invention, and particularly a step a) of trypsin treatment and a step b2) of SEC separation

Particularly, although only 6.25µg of purified samples were used the BBB model after 48h showed good permeation over BBB (basolateral side, in the target cells behind BBB) for all investigated samples of PPCs loaded with mRNA as a cargo molecule.

Particularly, it can be taken from Figure 9 that samples showed 1 to 3x 10^3 RLU after treatment for 48h when using 6.25µg of purified samples.

### Example 9: DLS confirms stable PPCs with antibody as cargo molecule and homogeneity of sample

As shown in Figure 10 a DLS measurement was conducted for samples containing PPCs with antibody as cargo molecule. Particularly, a DLS analysis showing the PDI of a mixture comprising a PPC loaded with an antibody as a cargo molecule and unloaded PPCs prior and after applying the method according to the present invention was performed. Thereby, the DLS analysis confirmed that PPCs loaded with antibody as cargo molecule are stable and a homogeneous mixture of antibody loaded PPCs is achieved after application of the method according to the present invention.

The respective DLS characteristics of samples before subjecting the mixture to a step a) of destabilization using trypsin as a destabilization agent, and after said step a), as well as a sample that was subsequently subjected to a step b2) of separation with SEC is depicted in Table 3 below.

As can be seen from the DLS curve characteristics in Figure 10, as well as from the characteristic values in Table 3, the PPCs loaded with antibody are stable after conducting the method steps a) and b2) according to the present invention. Thereby, homogeneity of the samples can be increased with subjecting the sample to step b2) of SEC separation. Furthermore, it is important to acknowledge that no free antibody is visible in the sample. This increased purity results from the advantageous trypsin treatment according to step a) of the inventive method, as free antibody and agglomerates are also advantageously digested by trypsin.

Accordingly, example 9 proves that PPCs loaded with antibodies as cargo molecules, are stable and may transduce cells after subjecting respective mixtures to the inventive method.

From Table 3 below it can be seen that all samples are in an adequate size range.

**Table 3: DLS characteristics of samples of PPC mixtures with antibody as cargo molecules**

| **Sample** | **Size (Volume) [nm]** | **Z-average [nm]** | **PDI** |
|---|---|---|---|
| w/o trypsin; w/o SEC | 32.67 | 57.18 | 0.143 |
| With trypsin; w/o SEC | 37.84 | 69.32 | 0.169 |
| With trypsin; with SEC | 43.82 | 60.49 | 0.149 |

### Example 10: Flow Cytometry analysis visualizing the attachment of antibodies VLPare loaded with antibodies to cells

In Example 10 flow cytometry analysis was conducted. In Figure 11 the flow cytometry profiles are shown of samples of mixtures of PPCs loaded with antibodies and before subjecting the mixture to the method according to the present invention, and particularly before subjecting the mixture to a step a) of destabilization with trypsin as a destabilization agent, and subsequently a step b2) of separation via SEC.

Figure 11 visualizes the attachment of antibodies PPCs are loaded with to cells using a mixture comprising a PPC loaded with an antibody as a cargo molecule and unloaded PPCs prior and after applying the method according to the present invention. Thereby, a successful loading and encapsulation and delivery of antibodies to cells could be proven by the shift of fluorescence signal of the different samples.

The inventors thereby have surprisingly found that also PPCs loaded with antibody are stable and further able to transduce cells after treatment with trypsin as a destabilization agent in a step a) of destabilization according to the present invention.

### Example 11: ELISA (Fig. 12A) and a qPCR (Fig. 12B), respectively, of brain samples of mice, and ex vivo Luciferase activity

In Example 11 mice were injected intravenously with 55 µg of PPCs loaded with mRNA as cargo molecule and without carrying out the method according to the present invention, i.e. without application of step a) and b2) and after carrying out the method according to the present invention, i.e. after application of step a) destabilizing with trypsin as destabilization agent and step b2) separation via SEC.

Figs. 12A and 12B show the results of an ELISA (Fig. 12A) and a qPCR (Fig. 12B), respectively, of brain samples taken from said mice previously injected with a mixture comprising PPCs loaded with mRNA as a cargo molecule and unloaded PPCs before and after application of the method according to the present invention as described above.

The mice were terminated 24 hours after injection and perfused with PBS. Brains were isolated and lysed for mRNA or protein assays. The mRNA was detected with qPCR, the result is shown in Fig. 12B. Detection of PPC protein VP1 was carried out with VP1-specific ELISA as shown in Fig. 12A.

As can be seen from Fig. 12B at least 3 times higher mRNA amount was found in samples treated with the method according to the present invention (left bar) compared to samples without such treatment (right bar).

As can be seen from Fig. 12A also 1,5-2 times higher VP1 protein amounts were detected in the brain samples treated with the method according to the present invention (two bars on the left, (SN -supernatant; P- pellet)) compared to samples without such treatment (two bars on the right).

Figure 13 shows that Luciferase activity can also be measured ex vivo in brain heads (ventral and dorsal) after application of PPCs subjected to the method according to the present invention.

### EMBODIMENTS

1. A method for purification of protein- or peptide-based capsules loaded with a cargo molecule from a mixture comprising said protein- or peptide-based capsules loaded with a cargo molecule and unloaded protein- or peptide-based capsules, the method comprising at least
   a step a) of subjecting the mixture to destabilization, preferably by adding a destabilizing agent and/or changing the physicochemical conditions, wherein the percentage of loaded protein- or peptide-based capsules is increased.
2. The method according to embodiment 1, wherein the method further comprises
   a step b1) of filtrating and/or
   a step b2) of separating.
3. The method according to embodiment 2, wherein the step b1) of filtrating is selected from the group comprising sterile filtration, dialysis, centrifugation, use of spin columns, in particular with immobilized nucleases and/or proteases, use of beads, in particular with subsequent pull-down assay and cross-flow filtration, preferably is sterile.
4. The method according to embodiment 2 or 3, wherein the step b2) of separating is selected from the group comprising filtration, ultrafiltration, e.g. dead-end filtration with MWCO, and/or cross flow filtration, chromatography, e.g. preparative chromatography, size-exclusion-chromatography (SEC), and/or affinity chromatography, and dynamic light scattering (DLS), preferably selected from the group comprising cross flow filtration and size-exclusion-chromatography (SEC).
5. The method according to any one of embodiments 1 to 4, wherein the destabilizing agent is a protease and/or a destabilization agent inducing a change of pH of the mixture.
6. The method according to any one of embodiments 1 to 5, wherein changing the physicochemical conditions is selected from the group comprising changing the physicochemical condition by mechanical treatment, particularly centrifugation or vortex, filtration with pressure, chromatography, particularly SEC with pressure, by change of temperature, particularly by heat denaturation, heat-shock treatment, preferably at temperatures of at maximum 96°C, or freeze-thaw treatment, by acoustic treatment, particularly with ultrasound, by osmotic treatment, particularly osmotic shock and pressure, by change of pH, particularly by adding acids or bases, and combinations thereof.
7. The method according to embodiment 5 or 6, wherein the protease is selected from the group comprising exopeptidases and endopeptidases, preferably the protease is selected from the group comprising aminopeptidase, dipeptidase, dipeptidyl-peptidase, peptidyl-dipeptidase, serin-carboxypeptidase, metallo-carboxypeptidase, cystein-carboypeptidase, omega-peptidase, serin-endopeptidase, cysteine-endopeptidase, aspartate-endopeptidase, metallo-endopeptidase, and threonine-endopeptidase, more preferably the protease is a serin-endopeptidase, most preferably the serin-endopeptidase is trypsin.
8. The method according to any one of the preceding embodiments, wherein before and/or after step b), the method additionally comprises a step of adding a nuclease, preferably selected from the group comprising endonuclease and exonuclease, more preferably selected from the group comprising DNAse and RNAse.
9. The method according to embodiment 8, wherein
   the nuclease is a DNAse, in cases where RNA, particularly mRNA, is selected as the cargo molecule; and/or
   the nuclease is an RNAse, in cases where DNA is selected as the cargo molecule and/or
   the nuclease is both DNAse and RNAse.
10. The method according to any of the preceding embodiments, wherein the protein- or peptide-based capsule comprises a capsid protein derived from a virus selected from the group comprising
   Parvoviridae, particularly adeno-associated virus;
   Retroviridae, particularly Lentivirus, such as HIV;
   Flaviviridae, particularly Hepatitis C virus;
   Hepadnaviridae, particularly Hepatitis B virus;
   Paramyxoviridae, particularly Nipah or measles virus;
   Polyomaviridae, particularly John Cunningham virus (JCV);
   Togaviridae, particularly Sindbis virus;
   Reoviridae, particularly rotavirus;
   Riboviridae, particularly Respiratory syncytial virus
   Picornaviridae, particularly foot-and-mouth-disease virus;
   Papillomaviridae, particularly human Papilloma virus;
   Caliciviridae, particularly Norwalk virus;
   Nodaviridae, particularly Flock House Virus;
   Virgaviridae, particularly Tobacco mosaic virus;
   bacteriophages, particularly Qβ, or AP205; and
   RNA phages.
11. The method according to any of the preceding embodiments, wherein the protein- or peptide-based capsule is present in the form of a virus-like particle (VLP).
12. The method according to any one of the preceding embodiments, wherein the protein- or peptide-based capsule, in particular the VLP, is capable of passing intact physiological and biological barriers, preferably blood brain barrier (BBB), in vivo.
13. The method according to any one of the preceding embodiments, wherein the cargo molecule comprises an RNA, a DNA, a protein or a combination thereof, a dye, a diagnostic agent, and/or an inorganic compound, preferably an mRNA and/or an antibody.
14. The method according to any one of the preceding embodiments, wherein the protein- or peptide-based capsule loaded with a cargo molecule fully incorporates the cargo molecule.
15. The method according to any one of the preceding embodiments, wherein the cargo molecule is a pharmaceutically acceptable product, preferably an active pharmaceutical ingredient (API).
16. A composition comprising protein- or peptide-based capsules loaded with a cargo molecule obtainable by the method according to any of the preceding embodiments.
17. The composition according to embodiment 16 for use in a method of *in vivo* diagnosis of a disease or disorder, preferably for the diagnosis of neurological disorders, in particular of CNS diseases, in a subject in need thereof.
18. The composition according to embodiment 17, wherein the neurological disorder is an LSD, Canavan disease and/or Krabbe disease.
19. The composition according to any of embodiments 16 to 18, for use in a method of *in vivo* diagnosis, wherein the protein- or peptide-based capsules are capable of passing intact physiological and biological barriers, particularly the blood brain barrier (BBB), particularly the physiologically intact BBB in vivo, and wherein preferably the crossing does not require a loss of integrity or increased permeability of the BBB.
20. The composition according to any one of embodiments 16 to 19 for use in a method of molecular imaging.
21. A Drug Delivery System (DDS) comprising protein- or peptide-based capsules loaded with a cargo molecule obtainable by the method according to any of embodiments 1 to 15.
22. The Drug Delivery System (DDS) according to embodiment 21 for use in a method of treatment and/or *in vivo* diagnosis of a disease or disorder, preferably for the treatment of neurological disorders, in particular of CNS diseases, in a subject in need thereof.
23. The Drug Delivery System (DDS) according to embodiment 22, wherein the neurological disorder is an LSD, Canavan disease and/or Krabbe disease.
24. The Drug Delivery System (DDS) according to any of embodiments 21 to 23, wherein the protein- or peptide-based capsules cross an intact physiological and biological barrier, particularly the blood brain barrier (BBB), particularly the physiologically intact BBB in vivo, and wherein more preferably the crossing does not require a loss of integrity or increased permeability of the BBB.
25. The Drug Delivery System (DDS) according to any one of embodiments 21 to 24, preferably comprising a VLP loaded with an mRNA or an antibody, for use in a method of treatment of a disease or disorder, preferably a neurological disorder, in particular a CNS disease.
26. Method of treatment of neurological disorders, in particular of CNS diseases, in a subject in need thereof, comprising a step of application of the Drug Delivery System (DDS) obtainable by a method comprising the method steps according to any of embodiments 1 to 15 to a subject in need thereof.
27. Method of *in vivo* diagnosis of neurological disorders, in particular of CNS diseases, in a subject in need thereof, comprising a step of application of the composition obtainable by a method comprising the method steps according to any of embodiments 1 to 15 to a subject in need thereof.

## Claims

1. A method for purification of protein- or peptide-based capsules loaded with a cargo molecule from a mixture comprising said protein- or peptide-based capsules loaded with a cargo molecule and unloaded protein- or peptide-based capsules, the method comprising at least
a step a) of subjecting the mixture to destabilization, preferably by adding a destabilizing agent and/or changing the physicochemical conditions, wherein the percentage of loaded protein- or peptide-based capsules is increased.

2. The method according to claim 1, wherein the method further comprises
a step b1) of filtrating and/or
a step b2) of separating.

3. The method according to claim 2, wherein the step b1) of filtrating is selected from the group comprising sterile filtration, dialysis, centrifugation, use of spin columns, in particular with immobilized nucleases and/or proteases, use of beads, in particular with subsequent pull-down assay and cross-flow filtration, preferably is sterile filtration.

4. The method according to claim 2 or 3, wherein the step b2) of separating is selected from the group comprising filtration, ultrafiltration, e.g. dead-end filtration with MWCO, and/or cross flow filtration, chromatography, e.g. preparative chromatography, size-exclusion-chromatography (SEC), and/or affinity chromatography, and dynamic light scattering (DLS), preferably selected from the group comprising cross flow filtration and size-exclusion-chromatography (SEC).

5. The method according to any one of claims 1 to 4, wherein the destabilizing agent is a protease and/or a destabilization agent inducing a change of pH of the mixture.

6. The method according to any one of claims 1 to 5, wherein changing the physicochemical conditions is selected from the group comprising changing the physicochemical condition by mechanical treatment, particularly centrifugation or vortex, filtration with pressure, chromatography, particularly SEC with pressure, by change of temperature, particularly by heat denaturation, heat-shock treatment, preferably at temperatures of at maximum 96°C, or freeze-thaw treatment, by acoustic treatment, particularly with ultrasound, by osmotic treatment, particularly osmotic shock and pressure, by change of pH, particularly by adding acids or bases, and combinations thereof.

7. The method according to any one of the preceding claims, wherein before and/or after step b), the method additionally comprises a step of adding a nuclease, preferably selected from the group comprising endonuclease and exonuclease, more preferably selected from the group comprising DNAse and RNAse.

8. The method according to any of the preceding claims, wherein the protein- or peptide-based capsule comprises a capsid protein derived from a virus selected from the group comprising
Parvoviridae, particularly adeno-associated virus;
Retroviridae, particularly Lentivirus, such as HIV;
Flaviviridae, particularly Hepatitis C virus;
Hepadnaviridae, particularly Hepatitis B virus;
Paramyxoviridae, particularly Nipah or measles virus;
Polyomaviridae, particularly John Cunningham virus (JCV);
Togaviridae, particularly Sindbis virus;
Reoviridae, particularly rotavirus;
Riboviridae, particularly Respiratory syncytial virus
Picornaviridae, particularly foot-and-mouth-disease virus;
Papillomaviridae, particularly human Papilloma virus;
Caliciviridae, particularly Norwalk virus;
Nodaviridae, particularly Flock House Virus;
Virgaviridae, particularly Tobacco mosaic virus;
bacteriophages, particularly Qβ, or AP205; and
RNA phages.

9. The method according to any of the preceding claims, wherein the protein- or peptide-based capsule is present in the form of a virus-like particle (VLP).

10. The method according to any one of the preceding claims, wherein the cargo molecule comprises an RNA, a DNA, a protein or a combination thereof, a dye, a diagnostic agent, and/or an inorganic compound, preferably an mRNA and/or an antibody.

11. The method according to any one of the preceding claims, wherein the cargo molecule is a pharmaceutically acceptable product, preferably an active pharmaceutical ingredient (API).

12. A composition comprising protein- or peptide-based capsules loaded with a cargo molecule obtainable by the method according to any of the preceding claims.

13. The composition according to claim 12 for use in a method of *in vivo* diagnosis of a disease or disorder, preferably for the diagnosis of neurological disorders, in particular of CNS diseases, preferably an LSD, Canavan disease and/or Krabbe disease, in a subject in need thereof.

14. A Drug Delivery System (DDS) comprising protein- or peptide-based capsules loaded with a cargo molecule obtainable by the method according to any of claims 1 to 11.

15. The Drug Delivery System (DDS) according to claim 14 for use in a method of treatment and/or *in vivo* diagnosis of a disease or disorder, preferably for the treatment of neurological disorders, in particular of CNS diseases, preferably an LSD, Canavan disease and/or Krabbe disease, in a subject in need thereof.
